# EUROPEAN PATENT SPECIFICATION

(11) **EP 4 561 692 B1**
(45) Date of publication and mention of the grant of the patent: **25.03.2026**
(21) Application number: 23750741.3
(22) Date of filing: 17.07.2023
(51) Int. Cl.: A61N 1/39, A61N 1/378, H02M 3/18

(54) **PREVENTING CAPACITOR REVERSE BIASING DURING DISCHARGE IN MEDICAL DEVICE**
VERHINDERUNG VON KONDENSATOR-UMKEHRVORSPANNUNG WÄHREND DER ENTLADUNG IN EINER MEDIZINISCHEN VORRICHTUNG
PRÉVENTION D'UNE POLARISATION INVERSE DE CONDENSATEUR PENDANT UNE DÉCHARGE DANS UN DISPOSITIF MÉDICAL

(30) Priority: 29.07.2022 US 202263369848 P
(43) Date of publication of application: 04.06.2025
(73) Proprietor: Medtronic, Inc., Minneapolis, Minnesota 55432 (US)
(72) Inventor: VISTE, Mark E., Minneapolis, Minnesota 55432 (US); NORTON, John D., Minneapolis, Minnesota 55432 (US)
(74) Representative: Zimmermann & Partner Patentanwälte mbB
(86) International application number: PCT/IB2023/057281
(87) International publication number: WO 2024/023632

(56) References cited:
- US-A- 5 391 186
- US-A- 5 405 361
- US-A- 6 096 063
- US-A1- 2020 206 519

## Description

### TECHNICAL FIELD

The disclosure relates to circuitry for delivery of cardiac therapy by implantable medical devices.

### BACKGROUND

For cardiac therapy (e.g., pacing pulses or defibrillation), a lead having one or more electrodes may be inserted on or near the heart, and an implantable medical device (IMD) may output electrical stimulation therapy. For leadless IMDs inserted on or near the heart, electrodes on the IMD output the electrical stimulation therapy. To avoid residual charge, the IMD may discharge the electrodes used to output the electrical stimulation therapy.

US2020/206519 Al relates to a method and device for delivering multi-phase defibrillation therapy.

### SUMMARY

In general, the disclosure describes example techniques to discharge capacitors used to deliver electrical stimulation therapy in a manner that prevents one or more capacitors from being reverse biased. For instance, processing circuitry may be configured to periodically interrupt the discharge of capacitors coupled in series, and recharge the capacitors in parallel to a common voltage. The processing circuitry may repeat the discharging and recharging to a common voltage until a final discharging where the voltage across the capacitors is at a desired, discharge voltage level.

In one example, the disclosure describes a medical system comprising therapy delivery circuitry comprising a plurality of capacitors, and processing circuitry configured to discharge the plurality of capacitors to a discharge voltage level, wherein to discharge the plurality of capacitors to the discharge voltage level, the processing circuitry is configured to repeatedly discharge the plurality of capacitors when coupled in series and partially recharge one or more capacitors of the plurality of capacitors when coupled in parallel until the voltage across the plurality of capacitors coupled in series is approximately equal to the discharge voltage level, wherein to discharge the plurality of capacitors, the processing circuitry is configured to discharge the plurality of capacitors when coupled in series to respective intermediate threshold voltage levels of a plurality of intermediate threshold voltage levels, and wherein to partially recharge the one or more capacitors, the processing circuitry is configured to, in response to a voltage across the plurality of capacitors coupled in series reaching the respective intermediate threshold voltage levels, charge the one or more capacitors of the plurality of capacitors to respective intermediate common voltage levels of a plurality of intermediate common voltage levels when coupled in parallel.

In one example, the disclosure describes a method for discharging capacitors, the method comprising discharging a plurality of capacitors to a discharge voltage level, wherein discharging the plurality of capacitors to the discharge voltage level comprises repeatedly discharging the plurality of capacitors when coupled in series and partially recharging one or more capacitors of the plurality of capacitors when coupled in parallel until a voltage across the plurality of capacitors coupled in series is approximately equal to the discharge voltage level, wherein discharging the plurality of capacitors comprises discharging the plurality of capacitors when coupled in series to respective intermediate threshold voltage levels of a plurality of intermediate threshold voltage levels, and partially recharging the one or more capacitors comprises, in response to a voltage across the plurality of capacitors coupled in series reaching the respective intermediate threshold voltage levels, charging the one or more capacitors of the plurality of capacitors to respective intermediate common voltage levels of a plurality of intermediate common voltage levels when coupled in parallel.

The details of one or more examples of the disclosure are set forth in the accompanying drawings and the description below. Other features, objects, and advantages of the disclosure will be apparent from the description and drawings, and from the claims.

### BRIEF DESCRIPTION OF DRAWINGS

FIG. 1A is a front view of a patient implanted with the extravascular ICD system implanted intra-thoracically.
FIG. 1B is a side view of the patient implanted with the extravascular ICD system implanted intra-thoracically.
FIG. 1C is a transverse view of the patient implanted with the extravascular ICD system implanted intra-thoracically.
FIG. 2 is a block diagram illustrating an example implantable medical device configured to perform one or more of the example techniques described in this disclosure.
FIGS. 3A-3C are circuit diagrams illustrating example circuits for discharging and partially charging capacitors in accordance with one or more examples described in this disclosure.
FIG. 4 is a flowchart illustrating examples of method of operations in accordance with one or more examples described in this disclosure.

### DETAILED DESCRIPTION

Therapy delivery circuitry for cardiac medical devices (e.g., pacemakers, defibrillators, etc.) is configured to output electrical stimulation therapy via one or more electrodes. The therapy delivery circuitry includes a plurality of capacitors coupled in series to deliver the electrical stimulation therapy to the heart. For example, to deliver therapy, a cardiac medical device includes a plurality of capacitors. The capacitors may be charged in parallel, and then coupled in series to deliver therapy such as defibrillation therapy. A capacitor includes two terminals (e.g., anode and cathode). In this disclosure, the plurality of capacitors being coupled in parallel means that the respective anode terminals are coupled together, and the respective cathode terminals are coupled together. The plurality of capacitors being coupled in series means that an anode terminal of a first capacitor is coupled to a cathode terminal of a second capacitor, and an anode terminal of the second capacitor is coupled to a cathode terminal of the third capacitor, and so forth.

For both parallel and series, the capacitors need not necessarily couple directly to one another. There may be resistors, diodes, etc. between the connections of the capacitors. In some examples, when coupled in parallel, there may be resistors, diodes, etc. between the connections of the capacitors, but when coupled in series, there may not be resistors, diodes, etc. between the connections of the capacitors.

As an example, if there are three capacitors, and each capacitor is charged in parallel to 260V, then when coupled in series, the voltage across the series of capacitors is 780V (e.g., 260*3). In some example cardiac medical devices, such as devices referred to as H-bridge pacing extravascular implantable cardiac device (EV-ICD), the capacitors used to deliver defibrillation therapy are the same capacitors used to deliver pacing therapy.

In some examples, after delivery of the defibrillation therapy, the voltage across the capacitors remains relatively high (e.g., in the order of hundreds of volts). Pacing therapy may be less effective if the pacing therapy is delivered with the voltage across the capacitors being relatively high. Accordingly, after delivery of the defibrillation therapy, there may be a discharge phase to discharge the voltage across the capacitors to a relatively low voltage level (e.g., less than 15V, such as less than 5V across each capacitor, and possibly close to 0V), referred to as a discharge voltage level. The example voltages are provided simply as examples, and should not be considered limiting. The specific voltages may be implementation dependent.

Ideally, each capacitor provides the same capacitance, and as the capacitors are discharged, the voltage across each capacitor drops equally so that the voltage across the series connection of the plurality of capacitors is at the discharge voltage level at the end of discharge. However, because capacitors cannot be manufactured to provide the precise capacitance, each capacitor may provide a different amount of capacitance within a tolerance (e.g., 450 µF within 7.5% tolerance). Due to different amount of capacitance, the discharge of the capacitors may not be even. For instance, for a discharge voltage level of zero, the voltage across the series connection of the plurality of capacitors may be near zero, but the voltage across any single capacitor may not be zero. Rather, the voltage across some capacitors may be positive, while the voltage across some capacitors may be negative. Voltage across capacitors being negative refers to the capacitors being reverse biased, which can damage electrolytic capacitors.

This disclosure describes examples of periodically interrupting discharge of capacitors connected in series at a plurality of voltage threshold levels (e.g., intermediate threshold voltages), recharging the capacitors to a common voltage in response to discharge reaching one of the plurality of voltage threshold levels, resuming discharging of the capacitors after recharge, and repeating the process until the voltage across the series of capacitors is approximately at the discharge voltage level (e.g., 0V in this example).

In one or more examples, there may be benefits in recharging in parallel. For instance, rather than selectively coupling capacitors to be recharged one at a time, by recharging in parallel, the example techniques provide for a way to level the voltages on each capacitor at one time.

For example, consider a capacitor with a nominal 450 µF capacitance, manufactured with an allowed variation of +/- 7.5 %. In this example, assume that three capacitors are connected in series. The worst case for voltage reversal would be one capacitor with the lowest possible capacitance (416 µF) and two with the highest possible capacitance (484 µF).

As an example, assume that the three capacitors are each charged to a voltage of 260V, such as by charging the three capacitors in parallel. The processing circuitry may then configure the three capacitors in series. In this example, the total voltage across the series connection of the three capacitors is 780V (e.g., 260V*3). In this example, assume that the three capacitors are to be discharged, such that the voltage across the three capacitors in series is 5V (e.g., the desired, discharge voltage level is 5V).

If the capacitance of each of the three capacitors is the same (e.g., each has capacitance of 450 µF), then after discharge to the discharge voltage level of 5V, the voltage across each capacitor would be the same and 5/3V (e.g., 5/3V + 5/3V + 5/3V = 5V). However, in this example, the capacitance of the three capacitors is not the same, but within the tolerance, where the capacitance of two capacitors is 484 µF (e.g., 450 µF + 7.5%) and the capacitance of one capacitor is 416 µF (e.g., 450 µF - 7.5%).

If the three capacitors were charged in parallel to 260V and then discharged in series to a combined 5V (e.g., the voltage across the series of capacitors is 5V), the low capacitance capacitor (e.g., 416 µF) would be expected to reach -25 V while the high capacitance capacitors would be expected to reach 15V (e.g., 484 µF), such that the three capacitors in series would be 5V. For instance, assume that the first capacitor has the lowest capacitance within tolerance and the second and third capacitors have the highest capacitance within tolerance. In this example, the voltage across the first capacitor would be -25V, while the voltage across each of the second and third capacitors would be 15V for a total voltage of 5V across the series of capacitors (e.g., 15V+15V+-25V = 5V).

In the above example, the voltage across one of the capacitors is described as being -25V. However, the capacitor may be incapable of being reverse biased to -25V. In some examples, a capacitor may be reverse biased to a -2V, and cannot be further reverse biased.

For electrolytic capacitors, undesired electrochemistry (e.g., chemical reaction within capacitor) would occur rather than reversal of the voltage to -25 V. For instance, there may be gassing or other processes within the capacitor that cause the capacitor to no longer provide the desired capacitance. Accordingly, in techniques described in this disclosure, instead of discharging from 260 V (for each single capacitor) to 5 V (for the set of capacitors in series), the processing circuitry may stop discharge at 75 V (for the set of capacitors in series), at which point the low capacitance member would be expected to be at +1 V, while the other two capacitors would each be expected to be at 37 V (e.g., 37+37+1 = 75V).

The processing circuitry may charge the capacitors to a common voltage such that after charging to the common voltage each capacitor is at the same voltage. For instance, the processing circuitry may charge each capacitor to 37V. However, since the two capacitors are already at 37V, only the third capacitor may be charged to 37V.

In some examples, rather than or in addition to charging the capacitors to the common voltage, the processing circuitry may couple the capacitors in parallel, but not provide any charge energy (e.g., not output a current through the capacitors). In such an example, the capacitors having the larger voltage may discharge into the capacitors having the smaller voltage until the voltages across the capacitors are substantially the same. In such examples, to limit the amount of charge that is flowing, the processing circuitry may couple resistors and/or diodes between the capacitors, thereby increasing the RC time constant. Inclusion of resistor and/or diodes between the capacitors may not be necessary in all examples.

The processing circuitry may then again discharge the capacitors. Taking the common voltage to be 37 V (the high single capacitor voltage), a discharge of these capacitors from 37 V (for each single capacitor) to 13 V (for the set of capacitors in series) again results in a low capacitance voltage of +1 V, this time with the high capacitance capacitors at 6V. Charging to a common 6V will allow discharge to 5V to be completed in the next step. In this example, a discharge from 780V for the set (260V for a single) to 5 V has been interrupted at 75V, 13V and 6V to restore the three capacitors to a common voltage. These intermediate voltages have been chosen such that the extreme mismatch would not result in reversal.

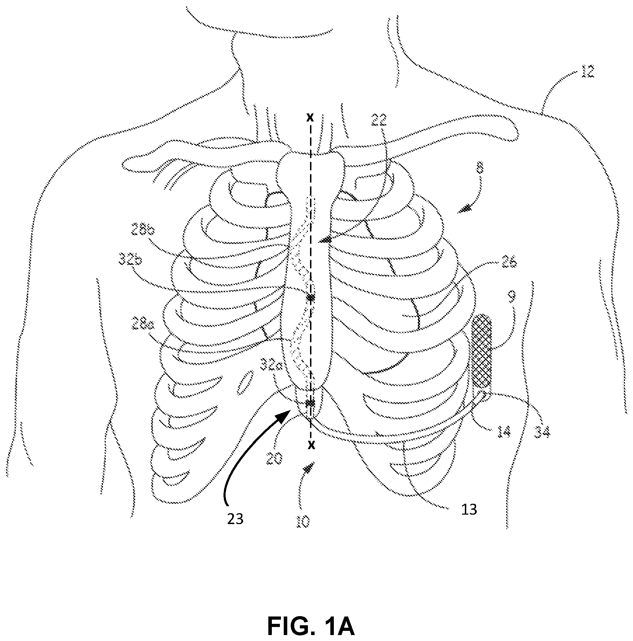

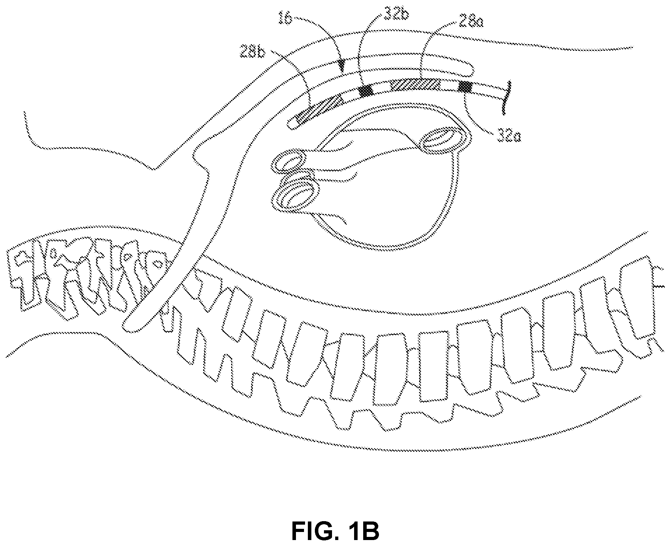

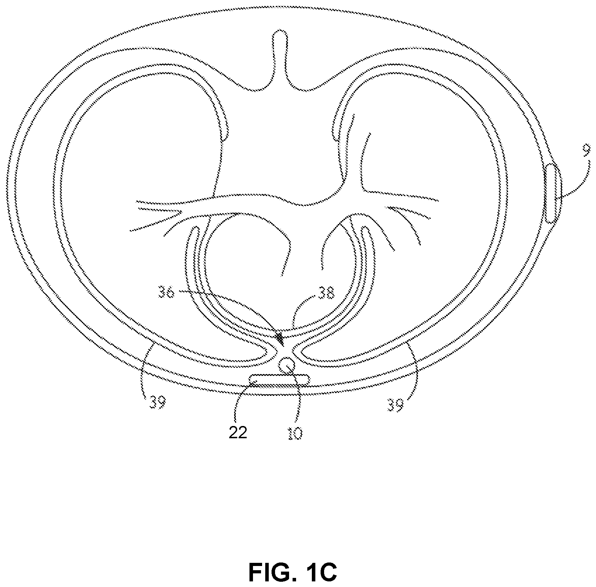

FIGS. 1A-1C illustrate different perspectives of extravascular implantable cardiac device (EV-ICD) system 8 implanted with a patient 12. FIG. 1A is a front view of a patient implanted with EV-ICD system 8. FIG. 1B is a side view of the patient implanted with EV-ICD system 8. FIG. 1C is a transverse view of the patient implanted with EV-ICD system 8. As illustrated, EV-ICD system 8 includes an ICD 9 connected to an implantable medical lead 10. ICD 9 and implantable medical lead 10 may be implanted intra-thoracically.

ICD 9 may include a housing that forms a hermetic seal that protects components of the ICD 9. The housing of ICD 9 may be formed of a conductive material, such as titanium or titanium alloy, which may function as a housing electrode (sometimes referred to as a can electrode). In some examples, ICD 9 may be formed to have or may include a plurality of electrodes on the housing. ICD 9 may also include a connector assembly (also referred to as a connector block or header) that includes electrical feedthroughs through which electrical connections are made between conductors of lead 10 and electronic components included within the housing of ICD 9. As will be described in further detail herein, the housing may house one or more processors, memories, transmitters, receivers, sensors, sensing circuitry, therapy circuitry, power sources and other appropriate components. The housing is configured to be implanted in a patient, such patient 12.

ICD 9 is implanted extra-thoracically on the left side of the patient, e.g., under the skin and outside the ribcage (subcutaneously or submuscularly). ICD 9 may, in some instances, be implanted between the left posterior axillary line and the left anterior axillary line of the patient. ICD 9 may, however, be implanted at other extra-thoracic locations on patient 12.

ICD 9 is illustrated and described as an example, and should not be considered limiting. The example techniques described in this disclosure may be applicable generally to implantable medical devices, and possibly other devices as well, that include discharging of capacitors to reduce residual charge within patient 12. For instance, the example techniques may be applicable to cardiac medical devices that are implanted intravascularly or implanted proximate to the heart. The example techniques may be applicable to other medical devices, such as implantable medical devices that provide stimulation to the spinal cord, to various nerves, and examples of implantable medical devices that provide deep brain stimulation (DBS).

Lead 10 may include an elongated lead body 13 having a distal portion 16 sized to be implanted in an extravascular location proximate the heart, e.g., intra-thoracically, as illustrated in FIGS. 1A-1C, or extra-thoracically. For example, lead 10 may extend extra-thoracically under the skin and outside the ribcage (e.g., subcutaneously or submuscularly) from ICD 9 toward the center of the torso of the patient, for example, toward the xiphoid process 23 of the patient. At a position proximate xiphoid process 23, the lead body 13 may bend or otherwise turn and extend superiorly. The bend may be pre-formed and/or lead body 13 may be flexible to facilitate bending. In the example illustrated in FIGS. 1A-1C, the lead body 13 extends superiorly intra-thoracically underneath the sternum, in a direction substantially parallel to the sternum.

Distal portion 16 of lead 10 may reside in a substernal location such that distal portion 16 of lead 10 extends superior along the posterior side of the sternum substantially within the anterior mediastinum 36. Anterior mediastinum 36 may be viewed as being bounded laterally by pleurae 39, posteriorly by pericardium 38, and anteriorly by the sternum 22. In some instances, the anterior wall of anterior mediastinum 36 may also be formed by the transversus thoracis and one or more costal cartilages. Anterior mediastinum 36 includes a quantity of loose connective tissue (such as areolar tissue), adipose tissue, some lymph vessels, lymph glands, substernal musculature (e.g., transverse thoracic muscle), the thymus gland, branches of the internal thoracic artery, and the internal thoracic vein (ITV).

Lead body 13 may extend superiorly extra-thoracically (instead of intra-thoracically), e.g., either subcutaneously or submuscularly above the ribcage/sternum. Lead 10 may be implanted at other locations, such as over the sternum, offset to the right of the sternum, angled lateral from the proximal or distal end of the sternum, or the like. In some examples, lead 10 may be implanted within an extracardiac vessel within the thorax, such as the ITV, the intercostal veins, the superior epigastric vein, or the azygos, hemiazygos, and accessory hemiazygos veins. In some examples, distal portion 16 of lead 10 may be oriented differently than is illustrated in FIGS. 1A-1C, such as orthogonal or otherwise transverse to sternum 22 and/or inferior to heart 26. In such examples, distal portion 16 of lead 10 may be at least partially within anterior mediastinum 36. In some examples, distal portion 16 of lead 10 may be placed between the heart and lung as well as within the pleural cavity.

Lead body 13 may have a generally tubular or cylindrical shape and may define a diameter of approximately 3-9 French (Fr). However, lead bodies of less than 3 Fr and more than 9 Fr may also be utilized. In another configuration, lead body 13 may have a flat, ribbon, or paddle shape with solid, woven filament, or metal mesh structure, along at least a portion of the length of the lead body 13. In such an example, the width across lead body 13 may be between 1-3.5 mm. Other lead body designs may be used without departing from the scope of this application.

Lead body 13 may be formed from a non-conductive material, including silicone, polyurethane, fluoropolymers, mixtures thereof, and other appropriate materials, and shaped to form one or more lumens (not shown), however, the techniques are not limited to such constructions. Distal portion 16 may be fabricated to be biased in a desired configuration, or alternatively, may be manipulated by the user into the desired configuration. For example, the distal portion 16 may be composed of a malleable material such that the user can manipulate the distal portion into a desired configuration where it remains until manipulated to a different configuration.

Lead body 13 may include a proximal end 14 and a distal portion 16 which include electrodes configured to deliver electrical energy to the heart or sense electrical signals of the heart. Distal portion 16 may be anchored to a desired position within the patient, for example, substernally or subcutaneously by, for example, suturing distal portion 16 to the patient's musculature, tissue, or bone at the xiphoid process entry site. In some examples, distal portion 16 may be anchored to the patient or through the use of rigid tines, prongs, barbs, clips, screws, and/or other projecting elements or flanges, disks, pliant tines, flaps, porous structures such as a mesh-like elements and metallic or non-metallic scaffolds that facilitate tissue growth for engagement, bio-adhesive surfaces, and/or any other non-piercing elements.

Lead body 13 may define a substantially linear portion 20 (FIG. 1A) as lead body 13 curves or bends near the xiphoid process 23 and extends superiorly. As shown in FIG. 1A, at least a part of distal portion 16 may define an undulating configuration distal to the substantially linear portion 20. In particular, distal portion 16 may define an undulating pattern, e.g., zig-zag, meandering, sinusoidal, serpentine, or other pattern, as it extends toward the distal end of lead 10. In other configurations, lead body 13 may not have a substantially linear portion 20 as it extends superiorly, but instead the undulating configuration may begin immediately after the bend.

Distal portion 16 includes one or more defibrillation electrodes configured to deliver an anti-tachyarrhythmia, e.g., cardioversion/defibrillation, shock to heart 26 of patient 12. In some examples, distal portion 16 includes a plurality of defibrillation electrodes spaced a distance apart from each other along the length of distal portion 16. In the example illustrated by FIGS. 1A-1C, distal portion 16 includes two defibrillation electrodes 28a and 28b (collectively, "defibrillation electrodes 28").

Defibrillation electrodes 28 may be disposed around or within the lead body 13 of the distal portion 16, or alternatively, may be embedded within the wall of the lead body 13. In one configuration, defibrillation electrodes 28 may be coil electrodes formed by a conductor. The conductor may be formed of one or more conductive polymers, ceramics, metal-polymer composites, semiconductors, metals or metal alloys, including but not limited to, one of a combination of the platinum, tantalum, titanium, niobium, zirconium, ruthenium, indium, gold, palladium, iron, zinc, silver, nickel, aluminum, molybdenum, stainless steel, MP35N, carbon, copper, polyaniline, polypyrrole, and other polymers. In another configuration, each of defibrillation electrodes 28 may be a flat ribbon electrode, a paddle electrode, a braided or woven electrode, a mesh electrode, a directional electrode, a patch electrode or another type of electrode configured to deliver a cardioversion/defibrillation shock to heart 26 of patient 12.

Defibrillation electrodes 28 may be electrically connected to one or more conductors, which may be disposed in the body wall of lead body 13 or in one or more insulated lumens (not shown) defined by lead body 13. In an example configuration, each of defibrillation electrodes 28 is connected to a common conductor such that a voltage may be applied simultaneously to all defibrillation electrodes 28 to deliver an anti-tachyarrhythmia shock to heart 26. In other configurations, defibrillation electrodes 28 may be attached to separate conductors such that each defibrillation electrode 28 may apply a voltage independent of the other defibrillation electrodes 28. In this case, ICD 9 or lead 10 may include one or more switches or other mechanisms to electrically connect the defibrillation electrodes together to function as a common polarity electrode such that a voltage may be applied simultaneously to all defibrillation electrodes 28 in addition to being able to independently apply a voltage.

Distal portion 16 may also include one or more pacing and/or sensing electrodes configured to deliver pacing pulses to heart 26 and/or sense electrical activity of heart 26. Such electrodes may be referred to as pacing electrodes, sensing electrodes, or pace/sense electrodes. In the example illustrated by FIGS. 1A-1C, distal portion 16 includes two pace/sense electrodes 32a and 32b (collectively, "pace/sense electrodes 32").

In the illustrated example of FIGS. 1A-1C, pace/sense electrode 32b is positioned between defibrillation electrodes 28, e.g., within a gap between the defibrillation electrodes, and pace/sense electrode 32a is positioned more proximal along distal portion 16 than proximal defibrillation electrode 28a. In some examples, more than one electrode 32 may exist within the gap between defibrillation electrodes 28. In some examples, an electrode 32 is additionally or alternatively located distal of the distalmost defibrillation electrode 28b.

Electrodes 32 may be configured to deliver low-voltage electrical pulses to the heart or may sense a cardiac electrical activity, e.g., depolarization and repolarization of the heart. As such, electrodes 32 may be referred to herein as pace/sense electrodes 32. In one configuration, electrodes 32 are ring electrodes. However, in other configurations electrodes 32 may be any of a number of different types of electrodes, including ring electrodes, short coil electrodes, paddle electrodes, hemispherical electrodes, or directional electrodes. Each of electrodes 32 may be the same or different types of electrodes as others of electrodes 32. Electrodes 32 may be electrically isolated from an adjacent defibrillation electrode 28 by including an electrically insulating layer of material between electrodes 32 and adjacent defibrillation electrodes 28. Each electrode 32 may have its own separate conductor such that a voltage may be applied to or sensed via each electrode independently from another electrode 32.

Electrodes 28 are referred to as defibrillation electrodes, and electrodes 32 are referred to as pace/sense electrodes, because they may have different physical structures enabling different functionality. Defibrillation electrodes 28 may be larger, e.g., have greater surface area, than pace/sense electrodes 32 and, consequently, may be configured to deliver anti-tachyarrhythmia shocks that have relatively higher voltages than pacing pulses. The relatively smaller size of pace/sense electrodes 32 may provide advantages over defibrillation electrodes for delivering pacing pulses and sensing intrinsic cardiac activity, e.g., lower pacing capture thresholds and/or better sensed signal quality. Nevertheless, a defibrillation electrode 28 may be used to deliver pacing pulses and/or sense electrical activity of the heart, such as in combination with a pace/sense electrode 32.

In the configuration shown in FIGS. 1A-1C, each electrode 32 is substantially aligned along a major longitudinal axis ("x"). In one example, the major longitudinal axis is defined by a portion of elongate body 12, e.g., substantially linear portion 20. In another example, the major longitudinal axis is defined relative to the body of the patient, e.g., along the anterior median line (or midsternal line), one of the sternal lines (or lateral sternal lines), left parasternal line, or other line.

In one configuration, the midpoint of each electrode 32a and 32b is along the major longitudinal axis "x," such that each electrode 32a and 32b is at least disposed at substantially the same horizontal position when the distal portion is implanted within the patient. In some examples, the longitudinal axis "x" may correspond to a caudal-cranial axis of the patient and a horizontal axis orthogonal to the longitudinal axis "x" may correspond to a medial-lateral axis of the patient. In other configurations, the electrodes 32 may be disposed at any longitudinal or horizontal position along the distal portion 16 disposed between, proximal to, or distal to the defibrillation electrodes 28. In the example illustrated in FIG. 1A, electrodes 32 are disposed along the undulating configuration of distal portion 16 at locations that will be closer to heart 26 of patient 12 than defibrillation electrodes 28 (e.g., at a peak of the undulating configuration that is toward the left side of the sternum). As illustrated in FIG. 1A, for example, electrodes 32 are substantially aligned with one another along the left sternal line. In the example illustrated in FIG. 1A, defibrillation electrodes 28 are disposed along peaks of the undulating configuration that extend toward a right side of the sternum away from the heart. This configuration places pace/sense electrodes 32 at locations closer to the heart than electrodes 28, to facilitate cardiac pacing and sensing at relatively lower amplitudes.

In some examples, pace/sense electrodes 32 and the defibrillation electrodes 28 may be disposed in a common plane when distal portion 16 is implanted extracardiovasculalry. In other configurations, the undulating configuration may not be substantially disposed in a common plane. For example, distal portion 16 may define a concavity or a curvature.

Proximal end 14 of lead body 13 may include one or more connectors 34 to electrically couple lead 10 to ICD 9. ICD 9 may also include a connector assembly that includes electrical feedthroughs through which electrical connections are made between the one or more connectors 34 of lead 10 and the electronic components included within the housing. The housing of ICD 9 may house one or more processors, memories, transmitters, receivers, sensors, sensing circuitry, therapy circuitry, power sources (e.g., capacitors and batteries), and/or other components. The components of ICD 9 may generate and deliver electrical therapy such as anti-tachycardia pacing, cardioversion or defibrillation shocks, post-shock pacing, and/or bradycardia pacing.

The undulating configuration of distal portion 16 and the inclusion of electrodes 32 between defibrillation electrodes 28 may provide a number of therapy vectors for the delivery of electrical therapy to the heart. For example, at least a portion of defibrillation electrodes 28 and one of electrodes 32 may be disposed over the right ventricle, or any chamber of the heart, such that pacing pulses and anti-tachyarrhythmia shocks may be delivered to the heart. The housing of ICD 9 may be charged with or function as a polarity different than the polarity of the one or more defibrillation electrodes 28 and/or electrodes 32 such that electrical energy may be delivered between the housing and the defibrillation electrode 28 and/or electrode 32 to the heart.

Each defibrillation electrode 28 may have the same polarity as every other defibrillation electrode 28 when a voltage is applied to it such that a shock may be delivered from all defibrillation electrodes together. In examples in which defibrillation electrodes 28 are electrically connected to a common conductor within lead body 13, this is the only configuration of defibrillation electrodes 28. However, in other examples, defibrillation electrodes 28 may be coupled to separate conductors within lead body 13 and may therefore each have different polarities such that electrical energy may flow between defibrillation electrodes 28, or between one of defibrillation electrodes 28 and one of pace/sense electrodes 32 or the housing electrode, to provide anti-tachyarrhythmia shock, pacing therapy, and/or to sense cardiac depolarizations. In this case, defibrillation electrodes 28 may still be electrically coupled together, e.g., via one or more switches within ICD 9, to have the same polarity.

In some examples, distal portion 16 of lead 10 may include one or more shields. The shield or shields may be configured to impede an electric field from delivery of an electrical therapy via an electrode, e.g., from a pacing pulse, in a direction from the electrode away from the heart, e.g., in an anterior direction. In this manner, the shield may reduce the likelihood that the electrical field will stimulate extracardiac tissue, such as sensory or motor nerves. Furthermore, the shield may direct the electrical field toward the heart, allowing lower energy level pacing pulses to capture the heart than may be required without the shield. Lower energy pacing pulses may also reduce the likelihood that pacing pulses delivered via the pacing electrode stimulate extracardiac tissue, and may result in less consumption of the power source of ICD 9 and, consequently, longer service life for the ICD. The techniques of this disclosure may be applied to implantable systems other than ICD 9, including, but not limited to, bradycardia pacemaker systems. For example, a lead that does not include defibrillation electrodes may include one or more shields and may be used with a pacemaker system without defibrillation capabilities.

As described above, ICD 9 may be configured to deliver defibrillation therapy via defibrillation electrodes 28 and pacing therapy via pace/sense electrodes 32. In some examples, to deliver defibrillation therapy or pacing therapy, therapy delivery circuitry of ICD 9 includes a plurality of capacitors. The processing circuitry of ICD 9 may be configured to charge the plurality of capacitors in parallel (e.g., the plurality of capacitors are coupled in parallel with one another). The plurality of capacitors being coupled in parallel means that the respective anode terminals are coupled together, and the respective cathode terminals are coupled together. In some examples, the respective anode and cathode terminals need not necessarily be coupled directly to one another, and may be coupled through resistors, diodes, etc., but inclusion of such resistors, diodes, etc. is not necessary in all examples.

Once charged in parallel, the processing circuitry of ICD 9 may couple the capacitors in series (e.g., a capacitor of the plurality of capacitors is coupled in series with a next capacitor in the plurality of capacitors). The plurality of capacitors being coupled in series means that an anode terminal of a first capacitor is coupled to a cathode terminal of a second capacitor, and an anode terminal of the second capacitor is coupled to an anode terminal of the third capacitor, and so forth. In some examples, there may be resistors, diodes, etc. between respective connections of an anode terminal to a cathode terminal, but inclusion of such resistors, diodes, etc. is not necessary in all examples.

When coupled in series, the processing circuitry may couple one end of the series of capacitors to one of defibrillation electrodes 28 or pace/sense electrodes 32, based on desired therapy, and couple other end of the series of capacitors to the housing of ICD 9, as one example. The result of such coupling may be a complete electrical path between defibrillation electrodes 28 or pace/sense electrodes 32, depending on desired therapy, and housing of ICD 9 and delivery of therapy.

As an example, a clinician or surgeon may use a programmer to program ICD 9. The programmer may present a display on which the clinician or surgeon may define an amount of Joules of energy that are to be delivered for defibrillation and/or pacing therapy. The programmer may transmit the information indicative of the amount of Joules. ICD 9 may store one or more look-up tables (LUTs) that indicate the voltage level for a given amount of Joules of therapy. The processing circuitry of ICD 9 may use the one or more LUTs to determine the voltage at which to charge the capacitors.

As an example, assume there are three capacitors, and the one or more LUTs indicate that the voltage level for the desired amount of Joules for defibrillation is 900V. In this example, the processing circuitry may charge the three capacitors in parallel to 300V, so that when the three capacitors are coupled in series, the voltage across the three capacitors is 900V.

The above example of using LUTs, information stored in LUTs, and the LUTs being on ICD 9 are all provided as an example, and the example techniques should not be considered limiting. In some examples, rather than LUTs, the processing circuitry of ICD 9 may utilize a mathematical function to determine the voltage. In some examples, the programmer may store the LUTs or may use the mathematical function to determine the voltage, and output information indicative of the voltage to ICD 9.

In some examples, the capacitors used to deliver defibrillation therapy and pacing therapy may be the same capacitors. For instance, in H-bridge pacing EV-ICDs, where the polarity of electrodes used to deliver the therapy can change, and possibly other types of EV-ICDs, ICDs in general, or other medical devices, the processing circuitry (e.g., processing circuitry of ICD 9) may utilize the capacitors to deliver defibrillation therapy, and then utilize the same capacitors to deliver pacing therapy. For instance, although there may be defibrillation electrodes 28 and pace/sense electrodes 32, in some examples, the same set of capacitors may couple to defibrillation electrodes 28 to deliver defibrillation therapy, and couple to pace/sense electrodes 32 to deliver pacing therapy.

For delivering defibrillation therapy, rather than the capacitors fully discharging to 0V, possibly after a mid-polarity switch, the capacitors may discharge to a relatively high voltage (e.g., 200V). For instance, the processing circuitry may charge the capacitors so that the voltage across the capacitors in series is approximately 780V, and after delivering a defibrillation therapy, the voltage across the capacitors in series may be approximately 200V. In this example, if there are three capacitors, the voltage across each capacitor may be approximately 66.67V (e.g., 200V/3).

However, having each of the capacitors at a relatively high voltage (e.g., at approximately 66.67V, in this example), and then transitioning to delivering pacing therapy may result in non-ideal pacing therapy. For instance, pacing therapy tends to use low voltage pulses. Accordingly, for pacing therapy, the voltage across the capacitors should be at a relatively low voltage at the start of the pacing therapy.

If the voltage across the capacitors is relatively high after a defibrillation pulse, it may be beneficial to discharge the capacitors to a discharge voltage level prior to beginning pacing therapy. Although the above example is described with respect to discharging the capacitors after a defibrillation pulse, there may be instances during delivery of pacing therapy where discharging of the capacitors to a discharge voltage level is beneficial, so that the pacing therapy can start from a relatively low voltage level.

One example way in which to discharge the plurality of capacitors is by coupling a current source to the plurality of capacitors that drains the charge built up on the capacitors. However, there may be issues when discharging the plurality of capacitors.

For instance, when the plurality of capacitors are coupled in series and are being discharged, if the capacitance of each of the capacitors is the same, then the voltage should drop at the same time and equally across the capacitors. As an example, if the discharge voltage level is 5V and there are three capacitors, then after discharging the voltage across the capacitors coupled in series, the voltage across each capacitor should be 5/3V (e.g., 5/3V+5/3V+5/3V = 5V).

However, capacitance of the capacitors tends not be exactly the same. Instead, the capacitance of the capacitors may be different but within an allowed variation. For instance, a capacitor with a nominal capacitance of 450 µF may vary ± 7.5% (e.g., between 416 µF and 484 µF). Due to imprecision of the capacitors, as well possibility of capacitance to drift over time, it may be possible that the capacitors used to deliver therapy range from the 484 µF to 416 µF. As one scenario, in examples where there are three capacitors, a first capacitor may be at 484 µF, a second capacitor may be at 484 µF, and a third capacitor may be at 416 µF.

In some examples, during discharge, the amount of charge (e.g., coulomb) per capacitor that is discharged may be the same. However, due to the variation in the capacitance, the voltage across each of the capacitors may not change (e.g., decrease) equally. For instance, the voltage across the capacitors when coupled in series may equal the discharge voltage level, but the voltage across each of the capacitors may not be equal. As an example, if a first and second capacitor is 484 µF, and a third capacitor is 416 µF, it may be possible that the voltage across a series coupling of the three capacitors is 5V (e.g., the summation of the voltage across each individual capacitor equals 5V). However, although the summation of the voltage across each individual capacitor equals 5V, it may not be necessarily true that the voltage across each individual capacitor is 5/3V. In this example, the voltage across the first capacitor and the second capacitor may be 15V, and the voltage across the third capacitor may be -25V (e.g., 15V + 15V + -25V = 5V). Therefore, the summation of the voltages is 5V, but the voltage across each of the capacitors is not 5/3V.

When the polarity of the voltage across a capacitor switches, the capacitor may be considered as being reverse biased. The above example where the voltage across the third capacitor is initially positive, but then switches to negative means that the third capacitor is reverse biased. In the above example where the third capacitor is reverse biased down to -25V is provided to ease with illustration. In general, it may be unlikely for a capacitor to reverse bias to -25V, and may not reverse bias below -2V.

Accordingly, in examples where a capacitor reverse biases, the voltage across the series coupling of the capacitors may not reach the discharge voltage level. Also, reverse biasing a capacitor may impact the capacitor. For instance, when the capacitor reverse biases there may be gassing of the capacitor, or other undesired electrochemistry reactions. In some examples, reverse biasing a capacitor can impact the amount of capacitance the capacitor provides.

This disclosure describes example techniques to discharge the plurality of capacitors used for therapy delivery in a way that minimizes or otherwise avoids reverse biasing a capacitor. For instance, as described in more detail, the processing circuitry may periodically interrupt the discharge of the capacitors, and partially recharge the capacitors to a common voltage level, and resume discharge.

In some examples, partially recharging the capacitors may include coupling the capacitors to a source (e.g., transformer) that delivers charge to the capacitors to recharge. However, the example techniques are not limited. Instead of or in addition to delivering charge to the capacitors, the processing circuitry may configure the capacitors to recharge one or more capacitors. For instance, when coupled in parallel, the even without addition recharge energy, the capacitors with the higher voltage may drain into the capacitors with the lower voltage, so that the voltages across the parallel coupling of the capacitors ends up approximately the same. In such examples, the capacitors with the lower voltage may be partially recharged from the charge on the capacitors with the higher voltage.

The processing circuitry may repeat such operations until the voltage across the capacitors reaches the discharge voltage level. For ease of illustration, the discharge voltage level is described as being the desired voltage level across the capacitors in series after discharge. However, the discharge voltage level may be measured as the voltage across any one of the capacitors or the capacitors coupled in parallel. That is, a system may define a desired voltage level across one capacitor. Even in such an example, the discharge voltage level may be equal to the defined voltage level multiplied by the number of capacitors to define the discharge voltage level across the capacitors coupled in series.

For instance, there may be a plurality of intermediate threshold voltage levels and a plurality of intermediate common voltage levels. During discharge, the processing circuitry may determine if the voltage across the capacitors coupled in series reached a first intermediate threshold voltage level. The first intermediate threshold level may be greater than the discharge voltage level.

If the voltage across the capacitors coupled in series reached the first intermediate threshold voltage level, the processing circuitry may couple the capacitors in parallel, and partially recharge one or more of the capacitors in parallel to a first intermediate common voltage level. In this disclosure, "partially recharge" includes examples where a source (e.g., transformer) delivers charge to partially recharge, and includes examples where the capacitors self-level by capacitors having higher voltage recharging capacitors having lower voltage without necessarily requiring a source to deliver charge. A combination of the techniques is also possible where some charge is delivered, and some amount of self-leveling occurs. That is, recharging or partially recharging includes charging one or more capacitors, such charging of the one or more capacitors may be done by coupling the one or more capacitors to source and/or by allowing the one or more capacitors to self-level.

The first intermediate common voltage level may be less than the voltage across each of the capacitors in parallel at the start of the discharge. Because the first intermediate common voltage level may be less than the voltage across each of the capacitors in parallel at the start of the discharge, the processing circuitry may be considered as "partially" recharging one or more capacitors of the plurality of capacitors. Also, in some examples, during the recharging, it may be possible that some of the capacitors do not need recharging. Therefore, the processing circuitry may selectively recharge only a subset of the capacitors (e.g., one or more capacitors of the plurality of capacitors).

In one or more examples, there may be benefits in recharging in parallel. For instance, the processing circuitry may be configured to level the voltages on each capacitor at one time (e.g., together) to respective intermediate common voltage levels, rather than individually recharging capacitors. Also, because the capacitors are coupled in parallel for recharging, there is a higher likelihood that the charge will be distribute such that the voltages across the capacitors are same. If the capacitors are recharged individually, there may be chances for deviation in the voltage levels of the capacitors resulting in possibility of reverse biased with discharged again.

After the recharge to the first intermediate common voltage level, the processing circuitry may discharge the capacitors in series. One example way to discharge the capacitors in series is by coupling to a current source, but the techniques are not so limited. The processing circuitry may determine if the voltage across the capacitors coupled in series reached a second intermediate threshold voltage level. The second intermediate threshold level may be greater than the discharge voltage level, and less than the first intermediate threshold voltage level.

If the voltage across the capacitors coupled in series reached the second intermediate threshold voltage level, the processing circuitry may couple the capacitors in parallel, and partially recharge one or more of the capacitors in parallel to a second intermediate common voltage level. One example way to charge the capacitors in parallel is by coupling to a transformer, but the techniques are not so limited, such as by self-leveling.

The second intermediate common voltage level may be less than the first intermediate common voltage. The processing circuitry may then repeat discharging to respective intermediate threshold voltage levels, and partially recharging to respective intermediate common voltage levels until the voltage across the capacitors coupled in series is approximately equal to the discharge voltage level (e.g., within ± 10% of the discharge voltage level).

For instance, to discharge the plurality of capacitors to the discharge voltage level, the processing circuitry may be configured to repeatedly discharge the plurality of capacitors when coupled in series and partially recharge one or more capacitors of the plurality of capacitors, followed by discharge, and then partial recharge until the voltage across the plurality of capacitors coupled in series is approximately equal to the discharge voltage level. To discharge the plurality of capacitors, the processing circuitry may be configured to discharge the plurality of capacitors when coupled in series to respective intermediate threshold voltage levels of a plurality of intermediate threshold voltage levels.

To partially recharge the one or more capacitors, the processing circuitry is configured to, in response to a voltage across the plurality of capacitors coupled in series reaching the respective intermediate threshold voltage levels, charge the one or more capacitors of the plurality of capacitors to respective intermediate common voltage levels of a plurality of intermediate common voltage levels when coupled in parallel. However, partially recharge may also include self-leveling where higher voltage capacitors recharge lower voltage capacitors when the capacitors are connected in parallel. In this way, to charge the one or more capacitors includes example where higher voltage capacitors charge lower voltage capacitors.

The capacitors coupled in series may be considered as a first circuit configuration, and the capacitors coupled in parallel may be considered as a second circuit configuration. In one or more examples, the processing circuitry may toggle the plurality of capacitors between the first circuit configuration and the second circuit configuration (e.g., based on reaching respective intermediate threshold voltage levels) until the voltage across the plurality of capacitors coupled in series is approximately equal to the discharge voltage level.

The voltage levels of the intermediate threshold voltage levels, and the voltage levels of the intermediate common voltage levels may be predefined based on the capacitance of the capacitors used to deliver therapy such that none of the capacitors reverse bias. For instance, consistent with the example provided above, assume there are three capacitors: first capacitor is at 484 µF, second capacitor is at 484 µF, and third capacitor is at 416 µF. In this example, assume that the voltage across the capacitors in series is 780V. The voltage of 780V is provided as an example, and in some examples, the voltage may be closer to 200V. Other voltage levels are also possible. Also, the discharge voltage level may be 5V.

In this example, a first intermediate threshold voltage level may be 75V, and a second intermediate threshold voltage level may be 13V. Also, in this example, a first intermediate common voltage level may be 37V, and a second intermediate common voltage level may be 6V. For instance, in this example, the plurality of capacitors may be in a first circuit configuration (e.g., in series), with a voltage of 780V across the series of capacitors. The processing circuitry of ICD 9 may couple a current source across the capacitors to start the discharge.

There may be various ways in which to determine the intermediate threshold voltage levels. For instance, the same amount of charge will be passed in each capacitor in series. The amount of charge that is passed (i.e., Q) is equal to the capacitance (i.e., C) multiplied by the voltage (i.e., V). Thus, Q=CV.

The discharge may need to be constrained by the smallest capacitance. Therefore, Q is determined by the smallest capacitance. For the smallest individual capacitance, Qmax = C(Vinit - Vmin), where Qmax is the maximum allowable charge passed, Vinit is the initial voltage, and Vmin is the minimum voltage (typically more than 0 V; maybe 1 V or other value to allow margin). For the overall stack of series capacitance, 1/Coverall = 1/C1 +1/C2+1/C3 ... for as many individual capacitors as there are. In this example, Vthreshhold = Vmax - Qmax/Coverall. The value of Vinit changes. For instance, Vinit may be the initial total voltage. Then, after reaching the first intermediate threshold voltage, Vinit may be equal to the first intermediate threshold voltage, and so forth.

The processing circuitry may determine discharge of the plurality of capacitors when coupled in series to a first intermediate threshold voltage level (e.g., 75V). The processing circuitry may then toggle the plurality of capacitors from the first circuit configuration to the second circuit configuration. For example, in response to discharging the plurality of capacitors when coupled in series to the first intermediate threshold voltage level (e.g., 75V), couple the plurality of capacitors in parallel. For instance, the processing circuitry may toggle the plurality of capacitors to the second circuit configuration.

In this example, based on the capacitance of the three capacitors (e.g., 484 µF, 484 µF, and 416 µF), the voltage across each of the first and second capacitors may be 37V, and the voltage across the third capacitor may be 1V (e.g., 37+37+1 = 75). Accordingly, in this example, the third capacitor is not yet reverse biased, but may be close to reverse biasing. In this example, the first intermediate threshold voltage level of 75V may be predefined based on the capacitance of the capacitors so that when the voltage reaches 75V, no capacitor is reverse biased. As described above, there may be equations to calculate the first intermediate threshold voltage level. In some examples, if individual capacitances are measured (e.g., in operation or prior to operation) and stored, it may be possible to dynamically determine the first intermediate threshold voltage using the above example equations.

With the capacitors coupled in parallel (e.g., in the second circuit configuration), the processing circuitry may recharge one or more capacitors to a first intermediate common voltage level (e.g., 37V). For example, the processing circuitry may couple the capacitors coupled in parallel to a transformer to recharge the capacitors. The transformer may be coupled to a control voltage source that switches current on and off through a first winding of the transformer causing a current on a second winding of the transformer, and a desired intermediate common voltage level across the capacitors in parallel.

However, in some examples, the processing circuitry may couple the capacitors in parallel, but not couple the capacitors to a source, like a transformer. In such examples, the capacitors may self-level to the same voltage, where the higher voltage capacitors charge, and thereby partially recharge, the lower voltage capacitors. The voltage level may be different than 37V in this example. For ease of illustration only, this disclosure describes examples with values for examples where a source, like a transformer, delivers charge to recharge the capacitors. However, the example techniques should not be considered limited.

In the above example, because the first and second capacitors are already at 37V, only the third capacitor may recharge to 37V. Also, in this example, the one or more capacitors may be considered as being "partially" recharged because the combined voltage across the three capacitors in series will be 111V (e.g., 37V+37V+37V = 111V), but the original voltage across the capacitors at the beginning of discharge was 780V.

In response to recharging the one or more capacitors of the plurality of capacitors to the first intermediate common voltage level, the processing circuitry may couple the plurality of capacitors in series. That is, the processing circuitry may toggle the capacitors from the second circuit configuration back to the first circuit configuration.

The processing circuitry may discharge the plurality of capacitors when coupled in series to a second intermediate threshold voltage level (e.g., 13V), such as by coupling to the current source. Similar to above, the second intermediate threshold voltage level may be predefined and stored, or may be calculated using the above example equations. In such examples, the Vinit may be 75V.

In this example, the voltage across the first capacitor and the second capacitor may be 6V, and the voltage across the third capacitor may be 1V. Similar to above, in this case, the third capacitor is not yet reverse biased. Accordingly, by setting the 13V as the second intermediate threshold voltage level, it may be possible to ensure that the third capacitor does not reverse bias.

In response to discharging the plurality of capacitors when coupled in series to the second intermediate threshold voltage level, the processing circuitry couple the plurality of capacitors in parallel. That is, the processing circuitry may toggle the capacitors from the first circuit configuration back to the second circuit configuration.

The processing circuitry may recharge the one or more capacitors of the plurality of capacitors to a second intermediate common voltage level (e.g., 6V), such as by coupling to the transformer and/or allowing capacitors to self-level. In this example, because the first and second capacitors are already at 6V, only the third capacitor may recharge to 6V. In examples of self-leveling, the capacitors may reach the same voltage, but may be less than 6V. Also, in this example, the one or more capacitors may be considered as being "partially" recharged because the combined voltage across the three capacitors in series will be 18V (e.g., 6V+6V+6V = 18V) or less, but the voltage across the capacitors was 111V when the discharge to 13V occurred. Accordingly, to charge the one or more capacitors may include use of a source to provide the charge or self-leveling.

In response to recharging the one or more capacitors of the plurality of capacitors to the second intermediate common voltage level (e.g., 6V), the processing circuitry may couple the plurality of capacitors in series. That is, the processing circuitry may toggle the capacitors from the second circuit configuration back to the first circuit configuration.

The processing circuitry discharges the plurality of capacitors when coupled in series to the discharge voltage level (e.g., 5V). The result of this process may be that the voltage across the capacitors in series is 5V, without any of the capacitors being reverse biased.

In the above example, the respective intermediate threshold voltage levels and intermediate common voltage levels may be predefined. However, the example techniques are not so limited. In some examples, the processing circuitry may include volt meters that measure the voltage across each of the capacitors. If the processing circuitry determines that the voltage across any one of the capacitors is below a threshold (e.g., less than 1V), the processing circuitry may interrupt the discharging, and couple the capacitors in parallel to rest the voltage level to an intermediate common voltage level. The intermediate common voltage level may not need to be predefined. The intermediate common voltage level may be the greatest voltage across the plurality of capacitors, as one example, but other examples are possible.

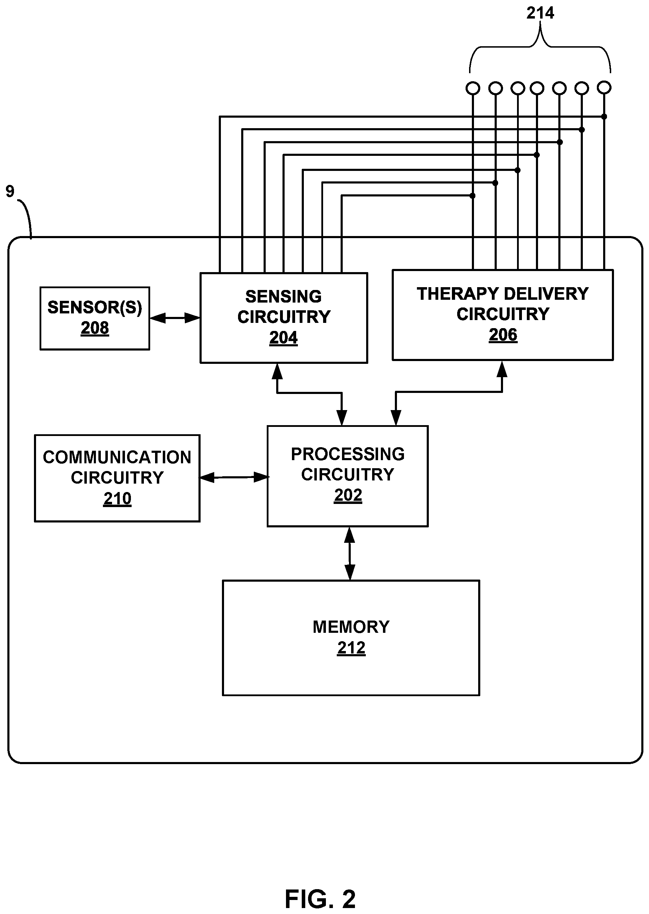

FIG. 2 is a functional block diagram of an example configuration of electronic components and other components of ICD 9. ICD 9 includes a processing circuitry 202, sensing circuitry 204, therapy delivery circuitry 206, sensors 208, communication circuitry 210, and memory 212. In some examples, ICD 9 may include more or fewer components. The described circuitry and other components may be implemented together on a common hardware component or separately as discrete but interoperable hardware or software components. Depiction of different features is intended to highlight different functional aspects and does not necessarily imply that such circuitry and other components must be realized by separate hardware or software components. Rather, functionality associated with one or more circuitries and components may be performed by separate hardware or software components, or integrated within common or separate hardware or software components.

Sensing circuitry 204 may be electrically coupled to some or all of electrodes 214, which may correspond to any of the defibrillation, pace/sense, and housing electrodes described herein. Sensing circuitry 204 is configured to obtain signals sensed via one or more combinations of electrodes 214 and process the obtained signals.

The components of sensing circuitry 204 may be analog components, digital components or a combination thereof. Sensing circuitry 204 may, for example, include one or more sense amplifiers, filters, rectifiers, threshold detectors, analog-to-digital converters (ADCs) or the like. Sensing circuitry 204 may convert the sensed signals to digital form and provide the digital signals to processing circuitry 202 for processing or analysis. For example, sensing circuitry 204 may amplify signals from the sensing electrodes and convert the amplified signals to multi-bit digital signals by an ADC. Sensing circuitry 204 may also compare processed signals to a threshold to detect the existence of atrial or ventricular depolarizations (e.g., P- or R waves) and indicate the existence of the atrial depolarization (e.g., P-waves) or ventricular depolarizations (e.g., R-waves) to processing circuitry 202. As shown in FIG. 2, ICD 9 may additionally include one or more sensors 208, such as one or more accelerometers, which may be configured to provide signals indicative of other parameters of a patient, such as activity or posture, to processing circuitry 202.

Processing circuitry 202 may process the signals from sensing circuitry 204 to monitor electrical activity of heart 26 of patient 12. Processing circuitry 202 may store signals obtained by sensing circuitry 204 as well as any generated EGM waveforms, marker channel data or other data derived based on the sensed signals in memory 212. Processing circuitry 202 may analyze the EGM waveforms and/or marker channel data to detect arrhythmias (e.g., bradycardia or tachycardia). In response to detecting the cardiac event, processing circuitry 202 may control therapy delivery circuitry 206 to deliver the desired therapy to treat the cardiac event, e.g., defibrillation shock, cardioversion shock, ATP, post shock pacing, or bradycardia pacing.

Therapy delivery circuitry 206 is configured to generate and deliver electrical therapy to heart 26. Therapy delivery circuitry 206 may include one or more pulse generators, capacitors, and/or other components capable of generating and/or storing energy to deliver as pacing therapy, defibrillation therapy, cardioversion therapy, cardiac resynchronization therapy, other therapy or a combination of therapies. In accordance with one or more examples described in this disclosure, processing circuitry 202 may discharge the plurality of capacitors coupled in series, but periodically interrupt the discharging of the capacitors and partially recharge one or more of the capacitors to ensure that there is not reverse biasing of the capacitors.

In some instances, therapy delivery circuitry 206 may include a first set of components configured to provide pacing therapy and a second set of components configured to provide defibrillation therapy. In some instances, therapy delivery circuitry 206 may utilize the same set of components to provide both pacing and defibrillation therapy. For example, the same plurality of capacitors may be configured to deliver defibrillation therapy and pacing therapy. In still other instances, therapy delivery circuitry 206 may share some of the defibrillation and pacing therapy components while using other components solely for defibrillation or pacing. Processing circuitry 202 may control therapy delivery circuitry 206 to deliver the generated therapy to heart 26 via one or more combinations of electrodes 214. Although not shown in FIG. 2, ICD 9 may include switching circuitry configurable by processing circuitry 202 to control which of electrodes 214 is connected to therapy delivery circuitry 206 and sensing circuitry 204.

Communication circuitry 210 may include any suitable hardware, firmware, software or any combination thereof for communicating with another device, such as a clinician programmer, a patient monitoring device, or the like. For example, communication circuitry 210 may include appropriate modulation, demodulation, frequency conversion, filtering, and amplifier components for transmission and reception of data with the aid of an antenna.

The various components of ICD 9 may include any one or more processors, controllers, digital signal processors (DSPs), application specific integrated circuits (ASICs), field-programmable gate arrays (FPGAs), or equivalent discrete or integrated circuitry, including analog circuitry, digital circuitry, or logic circuitry. Processing circuitry 202 may include fixed function circuitry and/or programmable processing circuitry. The functions attributed to processing circuitry 202 herein may be embodied as software, firmware, hardware or any combination thereof.

Memory 212 may include computer-readable instructions that, when executed by processing circuitry 202 or other components of ICD 9, cause one or more components of ICD 9 to perform various functions attributed to those components in this disclosure. Memory 212 may include any volatile, non-volatile, magnetic, optical, or electrical media, such as a random-access memory (RAM), read-only memory (ROM), non-volatile RAM (NVRAM), static non-volatile RAM (SRAM), electrically-erasable programmable ROM (EEPROM), flash memory, or any other non-transitory computer-readable storage media.

The leads and systems described herein may be used at least partially within the substernal space, e.g., within anterior mediastinum of patient, to provide an extravascular ICD system. An implanter (e.g., a surgeon) may implant the distal portion of the lead intra-thoracically using any of a number of implant tools, e.g., tunneling rod, sheath, or other tool that can traverse the diagrammatic attachments and form a tunnel in the substernal location. For example, the implanter may create an incision near the center of the torso of the patient, e.g., and introduce the implant tool into the substernal location via the incision. The implant tool is advanced from the incision superior along the posterior of the sternum in the substernal location. The distal portion of the lead is introduced into the tunnel via implant tool (e.g., via a sheath). As the distal portion is advanced through the substernal tunnel, the distal portion is relatively straight. The pre-formed or shaped undulating configuration is flexible enough to be straightened out while routing the lead through a sheath or other lumen or channel of the implant tool. Once the distal portion is in place, the implant tool is withdrawn toward the incision and removed from the body of the patient while leaving the lead in place along the substernal path. As the implant tool is withdrawn, the distal end of the lead takes on its pre-formed undulating configuration, and the shield transitions to its deployed configuration.

In some examples, rather than extending in a superior direction along the sternum, the distal portion of the lead may be oriented orthogonal or otherwise transverse to the sternum and/or inferior to the heart. In such examples, the lead may include one or more shields that cover a portion of an outer surface of one or more electrodes, e.g., an anterior and/or inferior portion, according to any of the examples described herein. Such shield(s) may impede an electrical field in a direction away from the heart, which may be an anterior and/or inferior direction. In some examples, the distal portion of the lead may be placed between the heart and lung as well as within the pleural cavity.

In accordance with one or more examples described in this disclosure, therapy delivery circuitry 206 may include a plurality of capacitors, and processing circuitry 202 may be configured to discharge the plurality of capacitors to a discharge voltage level. For example, to discharge the plurality of capacitors to the discharge voltage level, processing circuitry 202 may be configured to repeatedly discharge the plurality of capacitors when coupled in series and partially recharge one or more capacitors of the plurality of capacitors until the voltage across the plurality of capacitors coupled in series is approximately equal to the discharge voltage level. To discharge the plurality of capacitors, processing circuitry 202 may be configured to discharge the plurality of capacitors when coupled in series to respective intermediate threshold voltage levels of a plurality of intermediate threshold voltage levels, and to partially recharge the one or more capacitors, processing circuitry 202 may be configured to, in response to a voltage across the plurality of capacitors coupled in series reaching the respective intermediate threshold voltage levels, charge the one or more capacitors of the plurality of capacitors to respective intermediate common voltage levels of a plurality of intermediate common voltage levels when coupled in parallel.

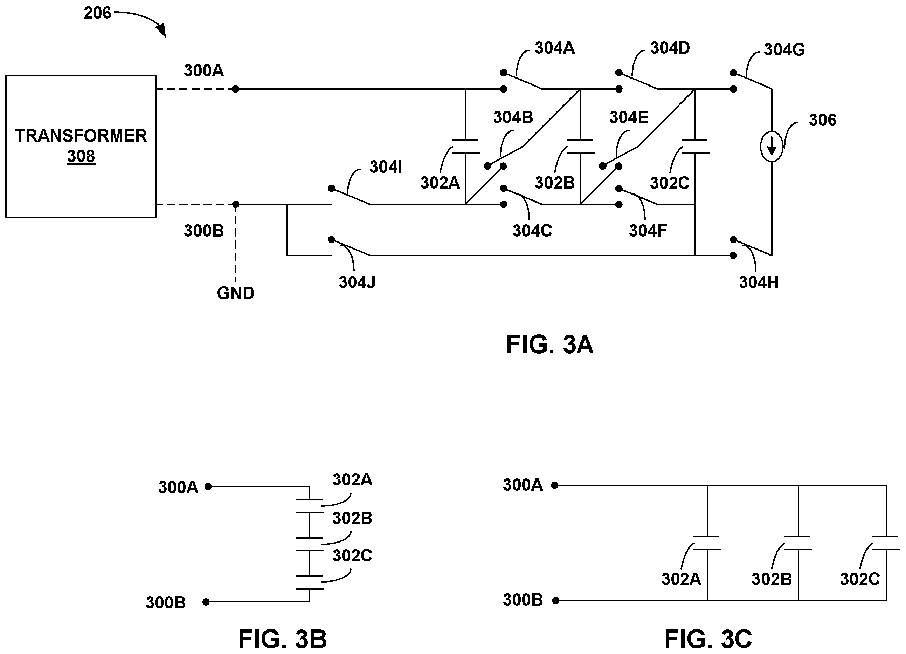

FIGS. 3A-3C are circuit diagrams illustrating example circuits for discharging and partially charging capacitors in accordance with one or more examples described in this disclosure. For ease of description, the capacitors and switches are described as being part of therapy delivery circuitry 206. However, the example techniques should not be considered so limiting.

As illustrated in FIG. 3A, therapy delivery circuitry 206 includes capacitors 302A, 302B, and 302C (collectively capacitors 302) and switches 304A-304J (collectively switches 304). Three capacitors 302 and ten switches 304 are illustrated simply as one example, and there may be more or fewer capacitors 302 and more or fewer switches 304. Also, the configuration of capacitors 302 and switches 304 is provided as an example and should not be considered limiting.

In one or more examples, processing circuitry 202 may determine the voltage across capacitors 302 when capacitors 302 are coupled in series. As one example, processing circuitry 302 may determine the voltage across nodes 300A and 300B when capacitors 302 are configured as illustrated in FIG. 3B (e.g., by closing switches 304B, 304E, and 304J.

To deliver therapy, processing circuitry 202 may close switches 304A, 304D, 304F, 304C, and 304I. Switches 304B, 304E, 304G, 304H, and 304J may be open. Processing circuitry 202 may couple transformer 308 to nodes 300A and 300B to charge capacitors 302 to a particular voltage level.

Once capacitors 302 are charged, processing circuitry 202 may close switches 304B, 304E, and 304J. Switches 304A, 304D, 304F, 304C, and 304I may be open. Processing circuitry 202 may couple node 300B to ground (e.g., housing of ICD 9). The result may be a complete circuit in which the capacitors discharge through tissue to ground, thereby delivering therapy.

In one or more examples, such as after a defibrillation pulse, including possible polarity switch of nodes 300A and 300B, processing circuitry 202 may be configured to stop the discharging of capacitors 302 at a particular voltage level (e.g., 200V), which may be relatively high.

In one or more techniques described in this disclosure, prior to switching from defibrillation therapy to pacing therapy, after the voltage across capacitors 302 when connected in series reaches a particular voltage level (e.g., 200V), processing circuitry 202 may toggle capacitors 302 in different circuit configurations to discharge the voltage across capacitors 302 to a discharge voltage level. Processing circuitry 202 may be configured to discharge plurality of capacitors 302 to a discharge voltage level. The discharge voltage level may be a relatively low voltage level, such as less than or equal to 5V or approximately equal to 0V (e.g., less than 1V).

To discharge plurality of capacitors to the discharge voltage level, processing circuitry 202 may be configured to repeatedly discharge plurality of capacitors 302 when coupled in series and partially recharge one or more capacitors of plurality of capacitors 302 until the voltage across the plurality of capacitors coupled in series is approximately equal to the discharge voltage level. For example, when switches 304B, 304E, and 304J are closed, capacitors 302 may form a first circuit configuration analogous to what is illustrated in FIG. 3B. For instance, in the first conceptual circuit configuration of FIG. 3B, capacitors 302 are in series. When switches 304A, 30D, 304F, 304C, and 304I are closed, capacitors 302 may form a second circuit configuration analogous to what is illustrated in FIG. 3C. For instance, in the second conceptual circuit configuration of FIG. 3C, capacitors 302 are in parallel.

To being the discharge to the discharge voltage level, processing circuitry 202 may be configured to couple plurality of capacitors 302 that are coupled in series (e.g., analogous to the first conceptual circuit configuration of FIG. 3B) to current source 306. For instance, processing circuitry 202 may disconnect nodes 300A and 300B from transformer 308, and close switches 304G and 304H when capacitors 302 are in the first circuit configuration (e.g., analogous to the first conceptual circuit configuration of FIG. 3B). As one example, current source 306 may source approximately (e.g., within manufacturing tolerance) 30 µA. However, other current levels are possible.

Processing circuitry 202 may discharge plurality of capacitors 302 when coupled in series to a first intermediate threshold voltage level. For instance, processing circuitry 202 may determine whether the voltage across nodes 300A and 300B is at the first intermediate threshold voltage level. There may be various ways in which processing circuitry 202 may determine whether the voltage across nodes 300A and 300B is at the first intermediate threshold voltage level.

As an example, processing circuitry 202 may measure the voltage across nodes 300A and 300B with a voltmeter to determine whether the voltage across nodes 300A and 300B is at the first intermediate threshold voltage level. As another example, processing circuitry 202 may measure amount of coulombs flowing through capacitors 302 (e.g., with a coulomb meter) to determine whether the voltage across nodes 300A and 300B is at the first intermediate threshold voltage level. As another example, the rate at which capacitors 302 discharge may be based on the amplitude of current source 306, capacitance of capacitors 302, and other factors such as a resistance. Processing circuitry 202 may determine whether the voltage across nodes 300A and 300B is at the first intermediate threshold voltage level based on amount of time that elapsed.

In response to discharging plurality of capacitors 302 when coupled in series to the first intermediate threshold voltage level, processing circuitry 202 may couple plurality of capacitors 302 in parallel (e.g., in the second circuit configuration of FIG. 3C). That is, processing circuitry 202 may toggle capacitors 302 from the first circuit configuration to the second circuit configuration. Processing circuitry 202 may recharge the one or more capacitors of plurality of capacitors 302 to a first intermediate common voltage level. For instance, processing circuitry 202 may disconnect current source 306 (e.g., open switches 304G and 304H), and couple nodes 300A and 300B to transformer 308 to recharge capacitors 302 in parallel. As another example, processing circuitry 202 may not couple nodes 300A and 300B to transformer 308. In such examples, capacitors 302 having the higher voltage may partially recharge the capacitors having the lower voltage. Also, in such examples, to limit current flow, the capacitors may be coupled to resistors and/or diodes during the partial recharge, but inclusion of resistors and/or diodes is not necessary in every example.

Processing circuitry 202 may repeat such operations. For example, processing circuitry 202 may be configured to repeatedly discharge plurality of capacitors 302 when coupled in series (e.g., in the first circuit configuration of FIG. 3B), and partially recharge one or more capacitors of the plurality of capacitors when coupled in parallel (e.g., analogous to the second conceptual circuit configuration of FIG. 3C) until the voltage across plurality of capacitors 302 coupled in series is approximately equal to the discharge voltage level. For instance, processing circuitry 202 may be configured to repeatedly toggle plurality of capacitors 302 (e.g., by closing and opening switches 304) between a first circuit configuration (e.g., analogous to FIG. 3B) and a second circuit configuration (e.g., analogous to FIG. 3C) until the voltage across plurality of capacitors 302 coupled in series is approximately equal to the discharge voltage level. As described, the first circuit configuration comprises capacitors 302 coupled in series (e.g., analogous to FIG. 3B), and the second circuit configuration comprises capacitors 302 coupled in parallel (e.g., analogous to FIG. 3C).

In one example, to discharge plurality of capacitors 302, processing circuitry 202 may be configured to discharge plurality of capacitors 302 when coupled in series (e.g., analogous to FIG. 3B) to respective intermediate threshold voltage levels of a plurality of intermediate threshold voltage levels. To partially recharge the one or more capacitors, processing circuitry 302 may be configured to, in response to a voltage across plurality of capacitors 302 coupled in series reaching the respective intermediate threshold voltage levels, charge the one or more capacitors of the plurality of capacitors to respective intermediate common voltage levels of a plurality of intermediate common voltage levels when coupled in parallel (e.g., analogous to FIG. 3C), or allow the one or more capacitors to self-level to respective intermediate common voltage levels.

In one or more examples, the respective intermediate common voltage levels for when transformer 308 delivers charge may be different than the respective intermediate common voltage levels for when capacitors 302 self-level. For ease, this disclosure refers to intermediate common voltage levels, but the intermediate common voltage levels may be at different levels based the partially recharge technique that is used.

To discharge plurality of capacitors 302, processing circuitry 202 may be configured to couple plurality of capacitors 302 that are coupled in series to current source 306. To partially recharge the one or more capacitors, processing circuitry 202 may be configured to couple plurality of capacitors 302 that are coupled in parallel to transformer 308 that is configured to recharge the one or more capacitors to the respective intermediate common voltage levels or to self-level to the respective intermediate common voltage levels.

There may be various ways in which processing circuitry 202 may determine that the voltage across capacitors 302 coupled in series (e.g., voltage across nodes 300A and 300B in FIG. 3B configuration) reached the respective intermediate threshold voltage levels of the plurality of intermediate threshold voltage levels. Processing circuitry 202 may be configured to determine that the voltage across capacitors 302 coupled in series reached the respective intermediate threshold voltage levels of the plurality of intermediate threshold voltage levels based at least on a determination of amount to time that elapsed. Processing circuitry 202 may be configured to determine that the voltage across capacitors 302 coupled in series reached the respective intermediate threshold voltage levels of the plurality of intermediate threshold voltage levels based at least on coulomb counting. Processing circuitry 202 may be configured to determine that the voltage across capacitors 302 coupled in series reached the respective intermediate threshold voltage levels of the plurality of intermediate threshold voltage levels based at least on a voltage measurement.

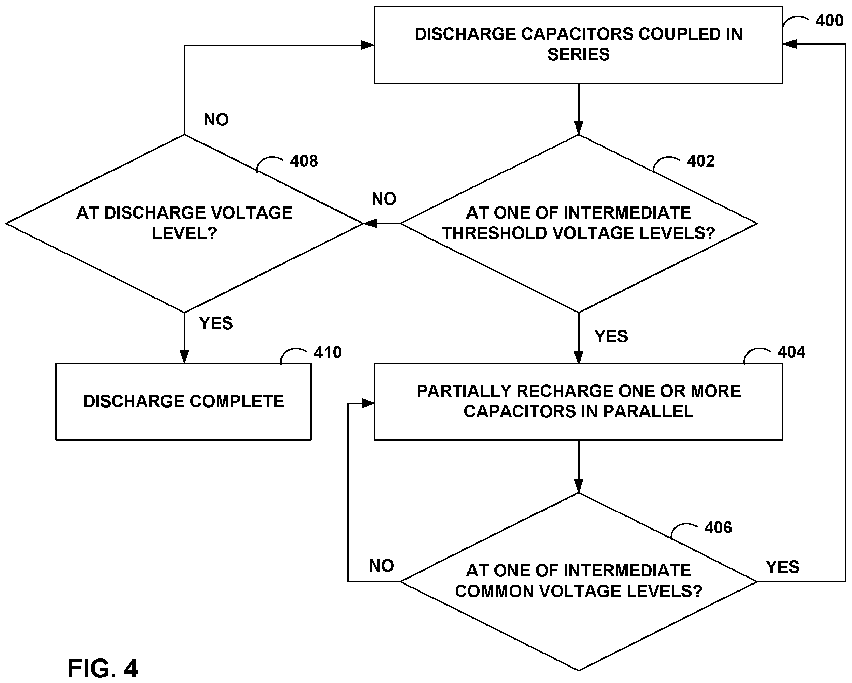

FIG. 4 is a flowchart illustrating examples of method of operations in accordance with one or more examples described in this disclosure. Processing circuitry 202 may discharge capacitors 302 coupled in series (400). For instance, processing circuitry 202 may close switches 304B, 304E, 304G, 304H, and 304J to couple capacitors 302 in series (e.g., a first circuit configuration analogous to what is shown in FIG. 3B) and couple current source 306 to discharge capacitors 302.

Processing circuitry 202 may determine whether voltage across capacitors 302 in series (e.g., voltage across nodes 300A and 300B in FIG. 3B) is at one of the intermediate threshold voltage levels (402). For instance, processing circuitry 202 may use a coulomb meter, voltmeter, or determine amount of elapsed time to determine whether voltage across capacitors 302 in series is at one of the intermediate threshold voltage levels. If not at one of the intermediate threshold voltage levels (NO of 402), processing circuitry 202 may determine whether the voltage across capacitors 302 in series is at the discharge voltage level (408). If not at the discharge voltage level (NO of 408), processing circuitry 202 may allow capacitors to keep discharging (400). If at the discharge voltage level (YES of 408), the discharge of capacitors 302 may be complete (410).

If processing circuit 202 determines that the voltage across capacitors 302 in series is at one of the intermediate threshold voltage levels (YES of 402), processing circuitry 202 may partially recharge one or more capacitors of capacitors 302 in parallel (404). For instance, processing circuitry 202 may close switches 304A, 304D, 304F, 304C, and 304I to couple capacitors 302 in parallel (e.g., a second circuit configuration analogous what is shown in FIG. 3C) and couple transformer 308 to partially recharge one or more capacitors of capacitors 302 in parallel. As another example, processing circuitry 202 may not necessarily couple capacitors 302 to transformer 308, and may allow capacitors 302 to self-level. In some examples, processing circuitry 202 may couple capacitors 302 but limit the amount of charge transformer 308 can deliver. In such examples, processing circuitry 202 may allow for a combination of recharging from transformer 308 and self-leveling.

Processing circuitry 202 may determine whether capacitors 302 coupled in parallel are at one of the intermediate common voltage levels (406). If capacitors 302 coupled in parallel are not at one of the intermediate common voltage levels (NO of 406), processing circuitry 202 may allow transformer 308 to keep recharging capacitors 302 coupled in parallel. If capacitors 302 coupled in parallel are at one of the intermediate common voltage levels (YES of 406), processing circuitry 202 may discharge capacitors 302 coupled in series (400).

In one or more examples, the functions described above may be implemented in hardware, software, firmware, or any combination thereof. If implemented in software, the functions may be stored on or transmitted over, as one or more instructions or code, a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

The term "non-transitory" may indicate that the storage medium is not embodied in a carrier wave or a propagated signal. In certain examples, a non-transitory storage medium may store data that can, over time, change (e.g., in RAM or cache). By way of example, and not limitation, such computer-readable storage media, may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer readable media. In some examples, an article of manufacture may include one or more computer-readable storage media.

Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Combinations of the above should also be included within the scope of computer-readable media.

Instructions may be executed by one or more processors, such as one or more DSPs, general purpose microprocessors, ASICs, FPGAs, or other equivalent integrated or discrete logic circuitry. Accordingly, the term "processor," as used herein, such as ECS controller 202, may refer to any of the foregoing structure or any other structure suitable for implementation of the techniques described herein. Also, the techniques could be fully implemented in one or more circuits or logic elements.

The techniques of this disclosure may be implemented in a wide variety of devices or apparatuses, including, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

## Claims

1. A medical system comprising:
therapy delivery circuitry (206) comprising a plurality of capacitors (302); and
processing circuitry (202) configured to discharge the plurality of capacitors to a discharge voltage level, wherein to discharge the plurality of capacitors to the discharge voltage level, the processing circuitry is configured to repeatedly discharge the plurality of capacitors when coupled in series and partially recharge one or more capacitors of the plurality of capacitors when coupled in parallel until the voltage across the plurality of capacitors coupled in series is approximately equal to the discharge voltage level,
wherein to discharge the plurality of capacitors, the processing circuitry is configured to discharge the plurality of capacitors when coupled in series to respective intermediate threshold voltage levels of a plurality of intermediate threshold voltage levels, and
wherein to partially recharge the one or more capacitors, the processing circuitry is configured to, in response to a voltage across the plurality of capacitors coupled in series reaching the respective intermediate threshold voltage levels, charge the one or more capacitors of the plurality of capacitors to respective intermediate common voltage levels of a plurality of intermediate common voltage levels when coupled in parallel.

2. The medical system of claim 1, wherein to repeatedly discharge the plurality of capacitors when coupled in series and partially recharge the one or more capacitors of the plurality of capacitors, the processing circuitry is configured to:
discharge the plurality of capacitors when coupled in series to a first intermediate threshold voltage level;
in response to discharging the plurality of capacitors when coupled in series to the first intermediate threshold voltage level, couple the plurality of capacitors in parallel;
recharge the one or more capacitors of the plurality of capacitors to a first intermediate common voltage level;
in response to recharging the one or more capacitors of the plurality of capacitors to the first intermediate common voltage level, couple the plurality of capacitors in series;
discharge the plurality of capacitors when coupled in series to a second intermediate threshold voltage level;
in response to discharging the plurality of capacitors when coupled in series to the second intermediate threshold voltage level, couple the plurality of capacitors in parallel;
recharge the one or more capacitors of the plurality of capacitors to a second intermediate common voltage level;
in response to recharging the one or more capacitors of the plurality of capacitors to the second intermediate common voltage level, couple the plurality of capacitors in series; and
discharge the plurality of capacitors when coupled in series to the discharge voltage level.

3. The medical system of any of claims 1 or 2, wherein to discharge the plurality of capacitors, the processing circuitry is configured to couple the plurality of capacitors that are coupled in series to a current source.

4. The medical system of any of claims 1-3, wherein to partially recharge the one or more capacitors, the processing circuitry is configured to couple the plurality of capacitors that are coupled in parallel to a transformer that is configured to recharge the one or more capacitors to the respective intermediate common voltage levels.

5. The medical system of any of claims 1-4, wherein to repeatedly discharge the plurality of capacitors when coupled in series and partially recharge the one or more capacitors of the plurality of capacitors, the processing circuitry is configured to repeatedly toggle the plurality of capacitors between a first circuit configuration and a second circuit configuration until the voltage across the plurality of capacitors coupled in series is approximately equal to the discharge voltage level,
wherein the first circuit configuration comprises the capacitors coupled in series, and
wherein the second circuit configuration comprises the capacitors coupled in parallel.

6. The medical system of any of claims 1-5, wherein the processing circuitry is configured to determine that the voltage across the capacitors coupled in series reached the respective intermediate threshold voltage levels of the plurality of intermediate threshold voltage levels based at least on one or more of a determination of amount to time that elapsed, on coulomb counting, or a voltage measurement.

7. The medical system of any of claims 1-6, further comprising a cardiac medical device (9), wherein the cardiac medical device comprises the therapy delivery circuitry and the processing circuitry.

8. The medical system of claim 7, wherein the cardiac medical device comprises an extravascular implantable cardiac device (EV-ICD).

9. The medical system of any of claims 1-8, wherein the same plurality of capacitors are configured to deliver defibrillation therapy and pacing therapy.

10. A method for preventing capacitor reverse biasing, the method comprising:
discharging a plurality of capacitors (302) to a discharge voltage level, wherein discharging the plurality of capacitors to the discharge voltage level comprises repeatedly discharging the plurality of capacitors when coupled in series and partially recharging one or more capacitors of the plurality of capacitors when coupled in parallel until a voltage across the plurality of capacitors coupled in series is approximately equal to the discharge voltage level,
wherein discharging the plurality of capacitors comprises discharging the plurality of capacitors when coupled in series to respective intermediate threshold voltage levels of a plurality of intermediate threshold voltage levels, and
partially recharging the one or more capacitors comprises, in response to a voltage across the plurality of capacitors coupled in series reaching the respective intermediate threshold voltage levels, charging the one or more capacitors of the plurality of capacitors to respective intermediate common voltage levels of a plurality of intermediate common voltage levels when coupled in parallel.

11. The method of claim 10, wherein repeatedly discharging the plurality of capacitors when coupled in series and partially recharging the one or more capacitors of the plurality of capacitors comprises:
discharging the plurality of capacitors when coupled in series to a first intermediate threshold voltage level;
in response to discharging the plurality of capacitors when coupled in series to the first intermediate threshold voltage level, coupling the plurality of capacitors in parallel;
recharging the one or more capacitors of the plurality of capacitors to a first intermediate common voltage level;
in response to recharging the one or more capacitors of the plurality of capacitors to the first intermediate common voltage level, coupling the plurality of capacitors in series;
discharging the plurality of capacitors when coupled in series to a second intermediate threshold voltage level;
in response to discharging the plurality of capacitors when coupled in series to the second intermediate threshold voltage level, coupling the plurality of capacitors in parallel;
recharging the one or more capacitors of the plurality of capacitors to a second intermediate common voltage level;
in response to recharging the one or more capacitors of the plurality of capacitors to the second intermediate common voltage level, coupling the plurality of capacitors in series; and
discharging the plurality of capacitors when coupled in series to the discharge voltage level.

12. The method any of claims 10 or 11, wherein partially recharging the one or more capacitors comprises coupling the plurality of capacitors that are coupled in parallel to a transformer that is configured to recharge the one or more capacitors to the respective intermediate common voltage levels.

13. The method of any of claims 10-12, wherein repeatedly discharging the plurality of capacitors when coupled in series and partially recharging the one or more capacitors of the plurality of capacitors comprises repeatedly toggling the plurality of capacitors between a first circuit configuration and a second circuit configuration until the voltage across the plurality of capacitors coupled in series is approximately equal to the discharge voltage level,
wherein the first circuit configuration comprises the capacitors coupled in series, and
wherein the second circuit configuration comprises the capacitors coupled in parallel.

14. The method of any of claims 10-13, further comprising determining that the voltage across the capacitors coupled in series reached the respective intermediate threshold voltage levels of the plurality of intermediate threshold voltage levels based at least on one or more of a determination of amount to time that elapsed, on coulomb counting, or a voltage measurement.

## Patentansprüche

1. Medizinisches System, umfassend:
Therapieabgabeschaltung (206) mit mehreren Kondensatoren (302); und
Verarbeitungsschaltung (202), die dazu ausgelegt ist, die mehreren Kondensatoren auf einen Entladespannungspegel zu entladen, wobei, zum Entladen der mehreren Kondensatoren auf den Entladespannungspegel, die Verarbeitungsschaltung dazu ausgelegt ist, die mehreren Kondensatoren wiederholt zu entladen, wenn sie in Reihe geschaltet sind, und einen oder mehrere Kondensatoren der Mehrzahl von Kondensatoren teilweise aufzuladen, wenn sie parallel geschaltet sind, bis die Spannung über die mehreren in Reihe geschalteten Kondensatoren annähernd gleich dem Entladespannungspegel ist;
wobei, zum Entladen der mehreren Kondensatoren, die Verarbeitungsschaltung dazu ausgelegt ist, die mehreren Kondensatoren, wenn sie in Reihe geschaltet sind, auf jeweilige Zwischenschwellenspannungspegel einer Mehrzahl von Zwischenschwellenspannungspegeln zu entladen, und
wobei, zum teilweisen Aufladen der ein oder mehreren Kondensatoren, die Verarbeitungsschaltung dazu ausgelegt ist, in Reaktion auf eine Spannung über die mehreren in Reihe geschalteten Kondensatoren, welche die jeweiligen Zwischenschwellenspannungspegel erreicht, die ein oder mehreren Kondensatoren der Mehrzahl von Kondensatoren auf jeweilige gemeinsame Zwischenspannungspegel einer Mehrzahl von gemeinsamen Zwischenspannungspegeln zu laden, wenn sie parallel geschaltet sind.

2. Medizinisches System nach Anspruch 1, wobei, um die mehreren Kondensatoren wiederholt zu entladen, wenn sie in Reihe geschaltet sind, und die ein oder mehreren Kondensatoren der Mehrzahl von Kondensatoren teilweise aufzuladen, die Verarbeitungsschaltung hierzu ausgelegt ist:
Entladen der mehreren Kondensatoren, wenn sie in Reihe geschaltet sind, auf einen ersten Zwischenschwellenspannungspegel;
in Reaktion auf das Entladen der mehreren Kondensatoren auf den ersten Zwischenschwellenspannungspegel, wenn sie in Reihe geschaltet sind, Parallelschalten der mehreren Kondensatoren;
Aufladen der ein oder mehreren Kondensatoren der Mehrzahl von Kondensatoren auf einen ersten gemeinsamen Zwischenspannungspegel;
in Reaktion auf das Aufladen der ein oder mehreren Kondensatoren der Mehrzahl von Kondensatoren auf den ersten gemeinsamen Zwischenspannungspegel, In-Reihe-Schalten der mehreren Kondensatoren;
Entladen der mehreren Kondensatoren, wenn sie in Reihe geschaltet sind, auf einen zweiten Zwischenschwellenspannungspegel;
in Reaktion auf das Entladen der mehreren Kondensatoren auf den zweiten Zwischenschwellenspannungspegel, wenn sie in Reihe geschaltet sind, Parallelschalten der mehreren Kondensatoren;
Aufladen der ein oder mehreren Kondensatoren der Mehrzahl von Kondensatoren auf einen zweiten gemeinsamen Zwischenspannungspegel;
in Reaktion auf das Aufladen der ein oder mehreren Kondensatoren der Mehrzahl von Kondensatoren auf den zweiten gemeinsamen Zwischenspannungspegel, In-Reihe-Schalten der mehreren Kondensatoren; und
Entladen der mehreren Kondensatoren, wenn sie in Reihe geschaltet sind, auf den Entladespannungspegel.

3. Medizinisches System nach einem der Ansprüche 1 oder 2, wobei, zum Entladen der mehreren Kondensatoren, die Verarbeitungsschaltung dazu ausgelegt ist, die mehreren Kondensatoren, die in Reihe geschaltet sind, an eine Stromquelle zu koppeln.

4. Medizinisches System nach einem der Ansprüche 1-3, wobei, zum teilweisen Aufladen der ein oder mehreren Kondensatoren, die Verarbeitungsschaltung dazu ausgelegt ist, die mehreren Kondensatoren, die parallel geschaltet sind, an einen Transformator zu koppeln, der dazu ausgelegt ist, die ein oder mehreren Kondensatoren auf die jeweiligen gemeinsamen Zwischenspannungspegel aufzuladen.

5. Medizinisches System nach einem der Ansprüche 1-4, wobei, zum wiederholten Entladen der mehreren Kondensatoren, wenn sie in Reihe geschaltet sind, und zum teilweisen Aufladen der ein oder mehreren Kondensatoren der Mehrzahl von Kondensatoren, die Verarbeitungsschaltung dazu ausgelegt ist, die mehreren Kondensatoren wiederholt zwischen einer ersten Schaltungskonfiguration und einer zweiten Schaltungskonfiguration umzuschalten, bis die Spannung über die mehreren in Reihe geschalteten Kondensatoren annähernd gleich dem Entladespannungspegel ist,
wobei die erste Schaltungskonfiguration die in Reihe geschalteten Kondensatoren umfasst, und
wobei die zweite Schaltungskonfiguration die parallel geschalteten Kondensatoren umfasst.

6. Medizinisches System nach einem der Ansprüche 1-5, wobei die Verarbeitungsschaltung dazu ausgelegt ist zu bestimmen, dass die Spannung über die in Reihe geschalteten Kondensatoren die jeweiligen Zwischenschwellenspannungspegel der Mehrzahl von Zwischenschwellenspannungspegeln erreicht hat, basierend auf wenigstens einem oder mehreren von einer Bestimmung der verstrichenen Zeit, einer Coulomb-Zählung oder einer Spannungsmessung.

7. Medizinisches System nach einem der Ansprüche 1-6, ferner umfassend eine kardiale medizinische Vorrichtung (9), wobei die kardiale medizinische Vorrichtung die Therapieabgabeschaltung und die Verarbeitungsschaltung umfasst.

8. Medizinisches System nach Anspruch 7, wobei die kardiale medizinische Vorrichtung eine extravaskuläre implantierbare kardiale Vorrichtung (EV-ICD) umfasst.

9. Medizinisches System nach einem der Ansprüche 1-8, wobei dieselben mehreren Kondensatoren dazu ausgelegt sind, eine Defibrillationstherapie und eine Stimulationstherapie abzugeben.

10. Verfahren zum Verhindern von Kondensator-Umkehrvorspannung, wobei das Verfahren umfasst:
Entladen mehrerer Kondensatoren (302) auf einen Entladespannungspegel, wobei das Entladen der mehreren Kondensatoren auf den Entladespannungspegel ein wiederholtes Entladen der mehreren Kondensatoren umfasst, wenn sie in Reihe geschaltet sind, und ein teilweises Aufladen eines oder mehrerer Kondensatoren der Mehrzahl von Kondensatoren, wenn sie parallel geschaltet sind, bis eine Spannung über die mehreren in Reihe geschalteten Kondensatoren annähernd gleich dem Entladespannungspegel ist;
wobei das Entladen der mehreren Kondensatoren ein Entladen der mehreren Kondensatoren, wenn sie in Reihe geschaltet sind, auf jeweilige Zwischenschwellenspannungspegel einer Mehrzahl von Zwischenschwellenspannungspegeln umfasst, und
teilweises Aufladen der ein oder mehreren Kondensatoren, in Reaktion darauf, dass eine Spannung über die mehreren in Reihe geschalteten Kondensatoren die jeweiligen Zwischenschwellenspannungspegel erreicht, Laden der ein oder mehreren Kondensatoren der Mehrzahl von Kondensatoren auf jeweilige gemeinsame Zwischenspannungspegel einer Mehrzahl von gemeinsamen Zwischenspannungspegeln, wenn sie parallel geschaltet sind.

11. Verfahren nach Anspruch 10, wobei ein wiederholtes Entladen der mehreren Kondensatoren, wenn diese in Reihe geschaltet sind, und ein teilweises Aufladen der ein oder mehreren Kondensatoren der Mehrzahl von Kondensatoren dies umfasst:
Entladen der mehreren Kondensatoren, wenn sie in Reihe geschaltet sind, auf einen ersten Zwischenschwellenspannungspegel;
in Reaktion auf das Entladen der mehreren Kondensatoren auf den ersten Zwischenschwellenspannungspegel, wenn sie in Reihe geschaltet sind, Parallelschalten der mehreren Kondensatoren;
Aufladen der ein oder mehreren Kondensatoren der Mehrzahl von Kondensatoren auf einen ersten gemeinsamen Zwischenspannungspegel;
in Reaktion auf das Aufladen der ein oder mehreren Kondensatoren der Mehrzahl von Kondensatoren auf den ersten gemeinsamen Zwischenspannungspegel, In-Reihe-Schalten der mehreren Kondensatoren;
Entladen der mehreren Kondensatoren, wenn sie in Reihe geschaltet sind, auf einen zweiten Zwischenschwellenspannungspegel;
in Reaktion auf das Entladen der mehreren Kondensatoren auf den zweiten Zwischenschwellenspannungspegel, wenn sie in Reihe geschaltet sind, Parallelschalten der mehreren Kondensatoren;
Aufladen der ein oder mehreren Kondensatoren der Mehrzahl von Kondensatoren auf einen zweiten gemeinsamen Zwischenspannungspegel;
in Reaktion auf das Aufladen der ein oder mehreren Kondensatoren der Mehrzahl von Kondensatoren auf den zweiten gemeinsamen Zwischenspannungspegel, In-Reihe-Schalten der mehreren Kondensatoren; und
Entladen der mehreren Kondensatoren, wenn sie in Reihe geschaltet sind, auf den Entladespannungspegel.

12. Verfahren nach einem der Ansprüche 10 oder 11, wobei ein teilweises Aufladen der ein oder mehreren Kondensatoren ein Koppeln der mehreren Kondensatoren, die parallel geschaltet sind, an einen Transformator umfasst, der dazu ausgelegt ist, die ein oder mehreren Kondensatoren auf die jeweiligen gemeinsamen Zwischenspannungspegel aufzuladen.

13. Verfahren nach einem der Ansprüche 10-12, wobei ein wiederholtes Entladen der mehreren Kondensatoren, wenn sie in Reihe geschaltet sind, und ein teilweises Aufladen der ein oder mehreren Kondensatoren der Mehrzahl von Kondensatoren, ein wiederholtes Umschalten der mehreren Kondensatoren zwischen einer ersten Schaltungskonfiguration und einer zweiten Schaltungskonfiguration umfasst, bis die Spannung über die mehreren in Reihe geschalteten Kondensatoren annähernd gleich dem Entladespannungspegel ist,
wobei die erste Schaltungskonfiguration die in Reihe geschalteten Kondensatoren umfasst, und
wobei die zweite Schaltungskonfiguration die parallel geschalteten Kondensatoren umfasst.

14. Verfahren nach einem der Ansprüche 10-13, ferner umfassend das Bestimmen, dass die Spannung über die in Reihe geschalteten Kondensatoren die jeweiligen Zwischenschwellenspannungspegel der mehreren Zwischenschwellenspannungspegel erreicht hat, basierend auf wenigstens einem oder mehreren von einer Bestimmung der verstrichenen Zeit, einer Coulomb-Zählung oder einer Spannungsmessung.

## Revendications

1. Système médical comprenant :
un circuit d'administration de thérapie (206) comprenant une pluralité de condensateurs (302) ; et
un circuit de traitement (202) configuré pour décharger la pluralité de condensateurs à un niveau de tension de décharge, pour décharger la pluralité de condensateurs au niveau de tension de décharge, le circuit de traitement étant configuré pour décharger de manière répétée la pluralité de condensateurs lorsqu'ils sont couplés en série et pour recharger partiellement un ou plusieurs condensateurs de la pluralité de condensateurs lorsqu'ils sont couplés en parallèle jusqu'à ce que la tension aux bornes de la pluralité de condensateurs couplés en série soit approximativement égale au niveau de tension de décharge,
pour décharger la pluralité de condensateurs, le circuit de traitement étant configuré pour décharger la pluralité de condensateurs lorsqu'il est couplé en série à des niveaux de tension de seuil intermédiaires respectifs d'une pluralité de niveaux de tension de seuil intermédiaires, et
pour recharger partiellement le ou les condensateurs, le circuit de traitement étant configuré pour, en réponse à une tension aux bornes de la pluralité de condensateurs couplés en série atteignant les niveaux de tension de seuil intermédiaires respectifs, charger le ou les condensateurs de la pluralité de condensateurs à des niveaux de tension communs intermédiaires respectifs d'une pluralité de niveaux de tension communs intermédiaires lorsqu'ils sont couplés en parallèle.

2. Système médical selon la revendication 1, pour décharger de manière répétée la pluralité de condensateurs lorsqu'ils sont couplés en série et recharger partiellement le ou les condensateurs de la pluralité de condensateurs, le circuit de traitement étant configuré pour :
décharger la pluralité de condensateurs lorsqu'ils sont couplés en série à un premier niveau de tension de seuil intermédiaire ;
en réponse à la décharge de la pluralité de condensateurs lorsqu'ils sont couplés en série au premier niveau de tension de seuil intermédiaire, coupler la pluralité de condensateurs en parallèle ;
recharger le ou les condensateurs de la pluralité de condensateurs à un premier niveau de tension commun intermédiaire ;
en réponse à la recharge du ou des condensateurs de la pluralité de condensateurs au premier niveau de tension commun intermédiaire, coupler la pluralité de condensateurs en série ;
décharger la pluralité de condensateurs lorsqu'ils sont couplés en série à un second niveau de tension de seuil intermédiaire ;
en réponse à la décharge de la pluralité de condensateurs lorsqu'ils sont couplés en série au second niveau de tension de seuil intermédiaire, coupler la pluralité de condensateurs en parallèle ;
recharger le ou les condensateurs de la pluralité de condensateurs à un second niveau de tension commun intermédiaire ;
en réponse à la recharge du ou des condensateurs de la pluralité de condensateurs au second niveau de tension commun intermédiaire, coupler la pluralité de condensateurs en série ; et
décharger la pluralité de condensateurs lorsqu'ils sont couplés en série au niveau de tension de décharge.

3. Système médical selon l'une quelconque des revendications 1 ou 2, pour décharger la pluralité de condensateurs, le circuit de traitement étant configuré pour coupler la pluralité de condensateurs qui sont couplés en série à une source de courant.

4. Système médical selon l'une quelconque des revendications 1 à 3, pour recharger partiellement le ou les condensateurs, le circuit de traitement étant configuré pour coupler la pluralité de condensateurs qui sont couplés en parallèle à un transformateur qui est configuré pour recharger le ou les condensateurs aux niveaux de tension communs intermédiaires respectifs.

5. Système médical selon l'une quelconque des revendications 1 à 4, pour décharger de manière répétée la pluralité de condensateurs lorsqu'ils sont couplés en série et recharger partiellement le ou les condensateurs de la pluralité de condensateurs, le circuit de traitement étant configuré pour basculer de manière répétée la pluralité de condensateurs entre une première configuration de circuit et une seconde configuration de circuit jusqu'à ce que la tension aux bornes de la pluralité de condensateurs couplés en série soit approximativement égale au niveau de tension de décharge, la première configuration de circuit comprenant les condensateurs couplés en série, et
la seconde configuration de circuit comprenant les condensateurs couplés en parallèle.

6. Système médical selon l'une quelconque des revendications 1 à 5, le circuit de traitement étant configuré pour déterminer que la tension aux bornes des condensateurs couplés en série a atteint les niveaux de tension de seuil intermédiaires respectifs de la pluralité de niveaux de tension de seuil intermédiaires sur la base d'au moins une ou plusieurs d'une détermination du temps écoulé, d'un comptage coulomb ou d'une mesure de tension.

7. Système médical selon l'une quelconque des revendications 1 à 6, comprenant en outre un dispositif médical cardiaque (9), le dispositif médical cardiaque comprenant le circuit d'administration de thérapie et le circuit de traitement.

8. Système médical selon la revendication 7, le dispositif médical cardiaque comprenant un dispositif cardiaque implantable extravasculaire (EV-ICD).

9. Système médical selon l'une quelconque des revendications 1 à 8, la même pluralité de condensateurs étant configurée pour administrer une thérapie de défibrillation et une thérapie de stimulation.

10. Procédé pour empêcher la polarisation inverse d'un condensateur, le procédé comprenant :
la décharge d'une pluralité de condensateurs (302) à un niveau de tension de décharge, la décharge de la pluralité de condensateurs au niveau de tension de décharge comprenant la décharge répétée de la pluralité de condensateurs lorsqu'ils sont couplés en série et la recharge partielle d'un ou plusieurs condensateurs de la pluralité de condensateurs lorsqu'ils sont couplés en parallèle jusqu'à ce qu'une tension aux bornes de la pluralité de condensateurs couplés en série soit approximativement égale au niveau de tension de décharge, la décharge de la pluralité de condensateurs comprenant la décharge de la pluralité de condensateurs lorsqu'ils sont couplés en série à des niveaux de tension de seuil intermédiaires respectifs d'une pluralité de niveaux de tension de seuil intermédiaires, et
la recharge partielle du ou des condensateurs comprenant, en réponse à une tension aux bornes de la pluralité de condensateurs couplés en série atteignant les niveaux de tension de seuil intermédiaires respectifs, la charge du ou des condensateurs de la pluralité de condensateurs à des niveaux de tension communs intermédiaires respectifs d'une pluralité de niveaux de tension communs intermédiaires lorsqu'ils sont couplés en parallèle.

11. Procédé selon la revendication 10, la décharge répétée de la pluralité de condensateurs lorsqu'ils sont couplés en série et la recharge partielle du ou des condensateurs de la pluralité de condensateurs comprenant :
la décharge de la pluralité de condensateurs lorsqu'ils sont couplés en série à un premier niveau de tension de seuil intermédiaire ;
en réponse à la décharge de la pluralité de condensateurs lorsqu'ils sont couplés en série au premier niveau de tension de seuil intermédiaire, le couplage de la pluralité de condensateurs en parallèle ;
la recharge du ou des condensateurs de la pluralité de condensateurs à un premier niveau de tension commun intermédiaire ;
en réponse à la recharge du ou des condensateurs de la pluralité de condensateurs au premier niveau de tension commun intermédiaire, le couplage de la pluralité de condensateurs en série ;
la décharge de la pluralité de condensateurs lorsqu'ils sont couplés en série à un second niveau de tension de seuil intermédiaire ;
en réponse à la décharge de la pluralité de condensateurs lorsqu'ils sont couplés en série au second niveau de tension de seuil intermédiaire, le couplage de la pluralité de condensateurs en parallèle ;
la recharge du ou des condensateurs de la pluralité de condensateurs à un second niveau de tension commun intermédiaire ;
en réponse à la recharge du ou des condensateurs de la pluralité de condensateurs au second niveau de tension commun intermédiaire, le couplage de la pluralité de condensateurs en série ; et
la décharge de la pluralité de condensateurs lorsqu'ils sont couplés en série au niveau de tension de décharge.

12. Procédé selon l'une quelconque des revendications 10 ou 11, la recharge partielle du ou des condensateurs comprenant le couplage de la pluralité de condensateurs qui sont couplés en parallèle à un transformateur qui est configuré pour recharger le ou les condensateurs aux niveaux de tension communs intermédiaires respectifs.

13. Procédé selon l'une quelconque des revendications 10 à 12, la décharge répétée de la pluralité de condensateurs lorsqu'ils sont couplés en série et la recharge partielle du ou des condensateurs de la pluralité de condensateurs comprenant le basculement répété de la pluralité de condensateurs entre une première configuration de circuit et une seconde configuration de circuit jusqu'à ce que la tension aux bornes de la pluralité de condensateurs couplés en série soit approximativement égale au niveau de tension de décharge.
la première configuration de circuit comprenant les condensateurs couplés en série, et
la seconde configuration de circuit comprenant les condensateurs couplés en parallèle.

14. Procédé selon l'une quelconque des revendications 10 à 13, comprenant en outre la détermination du fait que la tension aux bornes des condensateurs couplés en série a atteint les niveaux de tension de seuil intermédiaires respectifs de la pluralité de niveaux de tension de seuil intermédiaires sur la base d'au moins une ou plusieurs d'une détermination de la durée écoulée, d'un comptage coulomb ou d'une mesure de tension.
